# EUROPEAN PATENT SPECIFICATION

(11) **EP 1 945 661 B1**
(45) Date of publication and mention of the grant of the patent: **01.06.2011**
(21) Application number: 06794710.1
(22) Date of filing: 10.10.2006
(51) Int. Cl.: C07K 14/705, C12N 15/12, A61K 38/17, G01N 33/68, C12Q 1/68

(54) **GPR 146 RECEPTOR**
GPR-146-REZEPTOR
RÉCEPTEUR GPR 146

(30) Priority: 10.10.2005 GB 0520568
(43) Date of publication of application: 23.07.2008
(73) Proprietor: Takeda Pharmaceutical Company Limited, Osaka 541-0045 (JP)
(72) Inventor: GROSSE, Johannes, Cambridge CB4 0PA (GB); POWELL, Justin, Cambridge CB4 0PA (GB); DIXON, John, Cambridge CB4 0PA (GB); THRESHER, Rosemary, Cambridge CB4 0PA (GB); DAY, Kate, Cambridge CB4 0PA (GB)
(74) Representative: Jappy, John William Graham
(86) International application number: PCT/GB2006/003759
(87) International publication number: WO 2007/042792

(56) References cited:
- WO-A-2006/007400
- WO-A2-02/42458
- US-A1- 2005 014 689
- GLORIAM D E I ET AL: "Nine new human Rhodopsin family G-protein coupled receptors: identification, sequence characterisation and evolutionary relationship" BBA - GENERAL SUBJECTS, ELSEVIER SCIENCE PUBLISHERS, NL, vol. 1722, no. 3, 15 April 2005 (2005-04-15), pages 235-246, XP004783321 ISSN: 0304-4165

### SEQUENCE LISTING

<110> Paradigm Therapeutics Limited
<120> GPR146 Receptor
<130> REP07922WO
<150> GB0520568.7
   <151> 2005-10-10
<160> 20
<170> PatentIn version 3.3
<210> 1
   <211> 1853
   <212> DNA
   <213> Homo sapiens
<400> 1
   gtgcgcgccg tgagccccgc cgcctccgcc agcccgagct gcccgcccgg cggcgactgc     60
   gccggccgcc gcccagcaag ccgggtcgcg gagcctcgcc ggccgccatg tggagctgca    120
   gctggttcaa cggcacaggg ctggtggagg agctgcctgc ctgccaggac ctgcagctgg    180
   ggctgtcact gttgtcgctg ctgggcctgg tggtgggcgt gccagtgggc ctgtgctaca    240
   acgccctgct ggtgctggcc aacctacaca gcaaggccag catgaccatg ccggacgtgt    300
   actttgtcaa catggcagtg gcaggcctgg tgctcagcgc cctggcccct gtgcacctgc    360
   tcggcccccc gagctcccgg tgggcgctgt ggagtgtggg cggcgaagtc cacgtggcac    420
   tgcagatccc cttcaatgtg tcctcactgg tggccatgta ctccaccgcc ctgctgagcc    480
   tcgaccacta catcgagcgt gcactgccgc ggacctacat ggccagcgtg tacaacacgc    540
   ggcacgtgtg cggcttcgtg tggggtggcg cgctgctgac cagcttctcc tcgctgctct    600
   tctacatctg cagccatgtg tccacccgcg cgctagagtg cgccaagatg cagaacgcag    660
   aagctgccga cgccacgctg gtgttcatcg gctacgtggt gccagcactg gccaccctct    720
   acgcgctggt gctactctcc cgcgtccgca gggaggacac gcccctggac cgggacacgg    780
   gccggctgga gccctcggca cacaggctgc tggtggccac cgtgtgcacg cagtttgggc    840
   tctggacgcc acactatctg atcctgctgg ggcacacggt catcatctcg cgagggaagc    900
   ccgtggacgc acactacctg gggctactgc actttgtgaa ggatttctcc aaactcctgg    960
   ccttctccag cagctttgtg acaccacttc tctaccgcta catgaaccag agcttcccca   1020
   gcaagctcca acggctgatg aaaaagctgc cctgcgggga ccggcactgc tccccggacc   1080
   acatgggggt gcagcaggtg ctggcgtagg cggcccagcc ctcctgggga gacgtgactc   1140
   tggtggacgc agagcactta gttaccctgg acgctcccca catccttcca gaaggagacg   1200
   agctgctgga agagaagcag gaggggtgtt tttcttgaag tttccttttt cccacaaatg 1260
   ccactcttgg gccaaggctg tggtccccgt ggctggcatc tggcttgagt ctccccgagg 1320
   cctgtgcgtc tcccaaacac gcagctcaag gtccacatcc gcaaaagcct cctcgccttc 1380
   agcctcctca gcattcagtt tgtcaatgaa gtgatgaaag cttagagcca gtatttatac 1440
   tttgtggtta aaatacttga ttcccccttg tttgttttac aaaaacagat gtttcctaga 1500
   aaaatgacaa atagtaaaat gaacaaaacc ctacgaaaga atggcaacag ccagggtggc 1560
   cgggccctgc cagtgggcgg cgtgtgctag caaggcctgc cgggtgtgcc gcagtcacca 1620
   cagggttctg agaacatttc acagaagtgc ctgagacgcg gagacatggc tggtgttaaa 1680
   tggagctatt caatagcagt gacgcgctct cctcagccac caaatgtccc tgacaccctc 1740
   cccagccccc acagataaca tcagctgagg tttttttcag tatgaacctg tcctaaatca 1800
   attcctcaaa gtgtgcacaa aactaaagaa tataaataaa caaaagaaag gtg 1853
<210> 2
   <211> 1929
   <212> DNA
   <213> Homo sapiens
<400> 2
   ggccaggcgc gctgctgata agagcctgcg ggtttgcaga gaagcgcagc agcgcgcagg     60
   tcctcaccag agctctggtg gccacctctg tcccgccatg ctgctcaccg acagtggcca    120
   gggcccacag caccaagagg cttgggccac aaagtaaagg gtcgcggagc ctcgccggcc    180
   gccatgtgga gctgcagctg gttcaacggc acagggctgg tggaggagct gcctgcctgc    240
   caggacctgc agctggggct gtcactgttg tcgctgctgg gcctggtggt gggcgtgcca    300
   gtgggcctgt gctacaacgc cctgctggtg ctggccaacc tacacagcaa ggccagcatg    360
   accatgccgg acgtgtactt tgtcaacatg gcagtggcag gcctggtgct cagcgccctg    420
   gcccctgtgc acctgctcgg ccccccgagc tcccggtggg cgctgtggag tgtgggcggc    480
   gaagtccacg tggcactgca gatccccttc aatgtgtcct cactggtggc catgtactcc    540
   accgccctgc tgagcctcga ccactacatc gagcgtgcac tgccgcggac ctacatggcc    600
   agcgtgtaca acacgcggca cgtgtgcggc ttcgtgtggg gtggcgcgct gctgaccagc    660
   ttctcctcgc tgctcttcta catctgcagc catgtgtcca cccgcgcgct agagtgcgcc    720
   aagatgcaga acgcagaagc tgccgacgcc acgctggtgt tcatcggcta cgtggtgcca    780
   gcactggcca ccctctacgc gctggtgcta ctctcccgcg tccgcaggga ggacacgccc    840
   ctggaccggg acacgggccg gctggagccc tcggcacaca ggctgctggt ggccaccgtg    900
   tgcacgcagt ttgggctctg gacgccacac tatctgatcc tgctggggca cacggtcatc    960
   atctcgcgag ggaagcccgt ggacgcacac tacctggggc tactgcactt tgtgaaggat 1020
   ttctccaaac tcctggcctt ctccagcagc tttgtgacac cacttctcta ccgctacatg 1080
   aaccagagct tccccagcaa gctccaacgg ctgatgaaaa agctgccctg cggggaccgg 1140
   cactgctccc cggaccacat gggggtgcag caggtgctgg cgtaggcggc ccagccctcc 1200
   tggggagacg tgactctggt ggacgcagag cacttagtta ccctggacgc tccccacatc 1260
   cttccagaag gagacgagct gctggaagag aagcaggagg ggtgtttttc ttgaagtttc 1320
   ctttttccca caaatgccac tcttgggcca aggctgtggt ccccgtggct ggcatctggc 1380
   ttgagtctcc ccgaggcctg tgcgtctccc aaacacgcag ctcaaggtcc acatccgcaa 1440
   aagcctcctc gccttcagcc tcctcagcat tcagtttgtc aatgaagtga tgaaagctta 1500
   gagccagtat ttatactttg tggttaaaat acttgattcc cccttgtttg ttttacaaaa 1560
   acagatgttt cctagaaaaa tgacaaatag taaaatgaac aaaaccctac gaaagaatgg 1620
   caacagccag ggtggccggg ccctgccagt gggcggcgtg tgctagcaag gcctgccggg 1680
   tgtgccgcag tcaccacagg gttctgagaa catttcacag aagtgcctga gacgcggaga 1740
   catggctggt gttaaatgga gctattcaat agcagtgacg cgctctcctc agccaccaaa 1800
   tgtccctgac accctcccca gcccccacag ataacatcag ctgaggtttt tttcagtatg 1860
   aacctgtcct aaatcaattc ctcaaagtgt gcacaaaact aaagaatata aataaacaaa 1920
   agaaaggtg 1929
<210> 3
   <211> 1002
   <212> DNA
   <213> Homo sapiens
<400> 3
   atgtggagct gcagctggtt caacggcaca gggctggtgg aggagctgcc tgcctgccag 60
   gacctgcagc tggggctgtc actgttgtcg ctgctgggcc tggtggtggg cgtgccagtg 120
   ggcctgtgct acaacgccct gctggtgctg gccaacctac acagcaaggc cagcatgacc 180
   atgccggacg tgtactttgt caacatggca gtggcaggcc tggtgctcag cgccctggcc 240
   cctgtgcacc tgctcggccc cccgagctcc cggtgggcgc tgtggagtgt gggcggcgaa 300
   gtccacgtgg cactgcagat ccccttcaat gtgtcctcac tggtggccat gtactccacc 360
   gccctgctga gcctcgacca ctacatcgag cgtgcactgc cgcggaccta catggccagc 420
   gtgtacaaca cgcggcacgt gtgcggcttc gtgtggggtg gcgcgctgct gaccagcttc 480
   tcctcgctgc tcttctacat ctgcagccat gtgtccaccc gcgcgctaga gtgcgccaag 540
   atgcagaacg cagaagctgc cgacgccacg ctggtgttca tcggctacgt ggtgccagca 600
   ctggccaccc tctacgcgct ggtgctactc tcccgcgtcc gcagggagga cacgcccctg 660
   gaccgggaca cgggccggct ggagccctcg gcacacaggc tgctggtggc caccgtgtgc 720
   acgcagtttg ggctctggac gccacactat ctgatcctgc tggggcacac ggtcatcatc 780
   tcgcgaggga agcccgtgga cgcacactac ctggggctac tgcactttgt gaaggatttc 840
   tccaaactcc tggccttctc cagcagcttt gtgacaccac ttctctaccg ctacatgaac 900
   cagagcttcc ccagcaagct ccaacggctg atgaaaaagc tgccctgcgg ggaccggcac 960
   tgctccccgg accacatggg ggtgcagcag gtgctggcgt ag 1002
<210> 4
   <211> 334
   <212> PRT
   <213> Homo sapiens
<400> 4
   Met Trp Ser Cys Ser Trp Phe Asn Gly Thr Gly Leu Val Glu Glu Leu
   1               5                   10                  15
   Pro Ala Cys Gln Asp Leu Gln Leu Gly Leu Ser Leu Leu Ser Leu Leu
               20                  25                  30
   Gly Leu Val Val Gly Val Pro Val Gly Leu Cys Tyr Asn Ala Leu Leu
           35                  40                  45
   Val Leu Ala Asn Leu His Ser Lys Ala Ser Met Thr Met Pro Asp Val
       50                  55                  60
   Tyr Phe Val Asn Met Ala Val Ala Gly Leu Val Leu Ser Ala Leu Ala
   65                  70                  75                  80
   Pro Val His Leu Leu Gly Pro Pro Ser Ser Arg Trp Ala Leu Trp Ser
                   85                  90                  95
   Val Gly Gly Glu Val His Val Ala Leu Gln Ile Pro Phe Asn Val Ser
               100                 105                 110
   Ser Leu Val Ala Met Tyr Ser Thr Ala Leu Leu Ser Leu Asp His Tyr
           115                 120                 125
   Ile Glu Arg Ala Leu Pro Arg Thr Tyr Met Ala Ser Val Tyr Asn Thr
       130                 135                 140
   Arg His Val Cys Gly Phe Val Trp Gly Gly Ala Leu Leu Thr Ser Phe
   145                 150                 155                 160
   Ser Ser Leu Leu Phe Tyr Ile Cys Ser His Val Ser Thr Arg Ala Leu
                   165                 170                 175
   Glu Cys Ala Lys Met Gln Asn Ala Glu Ala Ala Asp Ala Thr Leu Val
               180                 185                 190
   Phe Ile Gly Tyr Val Val Pro Ala Leu Ala Thr Leu Tyr Ala Leu Val
           195                 200                 205
   Leu Leu Ser Arg Val Arg Arg Glu Asp Thr Pro Leu Asp Arg Asp Thr
       210                 215                 220
   Gly Arg Leu Glu Pro Ser Ala His Arg Leu Leu Val Ala Thr Val Cys
   225                 230                 235                 240
   Thr Gln Phe Gly Leu Trp Thr Pro His Tyr Leu Ile Leu Leu Gly His
                   245                 250                 255
   Thr Val Ile Ile Ser Arg Gly Lys Pro Val Asp Ala His Tyr Leu Gly
               260                 265                 270
   Leu Leu His Phe Val Lys Asp Phe Ser Lys Leu Leu Ala Phe Ser Ser
           275                 280                 285
   Ser Phe Val Thr Pro Leu Leu Tyr Arg Tyr Met Asn Gln Ser Phe Pro
       290                 295                 300
   Ser Lys Leu Gln Arg Leu Met Lys Lys Leu Pro Cys Gly Asp Arg His
   305                 310                 315                 320
   Cys Ser Pro Asp His Met Gly Val Gln Gln Val Leu Ala Glx
                   325                 330
<210> 5
   <211> 1002
   <212> DNA
   <213> Mus musculus
<400> 5
   atgtggagct gtggcccact caacagcaca gcgtgggctg aggagccgct gtgccggaac     60
   ctgcgcctgg ggctgtgggt cctctcgctg ctctacctgg gggcaggggt ccctgtgagc    120
   ttaggctata atgctcttct ggtgctggcc aacctggcca gcaagaacac catgaccatg    180
   cccgacgtgt acttcgtgaa catggccgtg gcggggctgg tgctcacggc actggcacct    240
   gcgtacctgt tgggccctgc ccactccagg tgggccctgt ggagcctcag cagtgaggcc    300
   catgtgacac tgctcatcct gttcaacgtg gcttccctgg tgaccatgta ctccactgca    360
   ctgctgagcc ttgactacta catcgagcgt gccctgccac gcacctacat ggccagtgtg    420
   tacaacaccc ggcacgtgtg tggcttcgtc tggggagggg cggtgctcac cagcttctcc    480
   tccctgctct tctacatctg cagtcacgtg tcttctagaa tcgctgagtg tgcccggatg    540
   cagaacacgg aggcagccga tgctatcctt gtgctcatcg gctacgtggt gccaggtctg    600
   gctgtgttgt atgccctggc actcatctcg agaatcggga aggaagacac acccctggac    660
   caggacacca gcaggctgga cccctcggtg cacaggctgc tggtggccac cgtgtgcact    720
   cagtttggcc tctggacacc ttactacttg agcctggggc acacagtgct gacgtcacgg    780
   gggaggaccg tggaggggca ttatctgggc atcctacagg ttgctaagga cctggccaag    840
   ttcctggcct tctcaagcag ttctgtgaca ccactgctct accgttacat caacaaagcc    900
   ttccccggca agctccggcg gctgatgaag aagatgcact gcgggcgccg ccactgctcc    960
   cccgacccct cggggataca gcaggtgatg gcacaggcgt ag                      1002
<210> 6
   <211> 334
   <212> PRT
   <213> Mus musculus
<400> 6
   Met Trp Ser Cys Gly Pro Leu Asn Ser Thr Ala Trp Ala Glu Glu Pro
   1               5                   10                  15
   Leu Cys Arg Asn Leu Arg Leu Gly Leu Trp Val Leu Ser Leu Leu Tyr
               20                  25                  30
   Leu Gly Ala Gly Val Pro Val Ser Leu Gly Tyr Asn Ala Leu Leu Val
           35                  40                  45
   Leu Ala Asn Leu Ala Ser Lys Asn Thr Met Thr Met Pro Asp Val Tyr
       50                  55                  60
   Phe Val Asn Met Ala Val Ala Gly Leu Val Leu Thr Ala Leu Ala Pro
   65                  70                  75                  80
   Ala Tyr Leu Leu Gly Pro Ala His Ser Arg Trp Ala Leu Trp Ser Leu
                   85                  90                  95
   Ser Ser Glu Ala His Val Thr Leu Leu Ile Leu Phe Asn Val Ala Ser
               100                 105                 110
   Leu Val Thr Met Tyr Ser Thr Ala Leu Leu Ser Leu Asp Tyr Tyr Ile
   115 120 125
   Glu Arg Ala Leu Pro Arg Thr Tyr Met Ala Ser Val Tyr Asn Thr Arg
   130 135 140
   His Val Cys Gly Phe Val Trp Gly Gly Ala Val Leu Thr Ser Phe Ser
   145 150 155 160
   Ser Leu Leu Phe Tyr Ile Cys Ser His Val Ser Ser Arg Ile Ala Glu
   165 170 175
   Cys Ala Arg Met Gln Asn Thr Glu Ala Ala Asp Ala Ile Leu Val Leu
   180 185 190
   Ile Gly Tyr Val Val Pro Gly Leu Ala Val Leu Tyr Ala Leu Ala Leu
   195 200 205
   Ile Ser Arg Ile Gly Lys Glu Asp Thr Pro Leu Asp Gln Asp Thr Ser
   210 215 220
   Arg Leu Asp Pro Ser Val His Arg Leu Leu Val Ala Thr Val Cys Thr
   225 230 235 240
   Gln Phe Gly Leu Trp Thr Pro Tyr Tyr Leu Ser Leu Gly His Thr Val
   245 250 255
   Leu Thr Ser Arg Gly Arg Thr Val Glu Gly His Tyr Leu Gly Ile Leu
   260 265 270
   Gln Val Ala Lys Asp Leu Ala Lys Phe Leu Ala Phe Ser Ser Ser Ser
   275 280 285
   Val Thr Pro Leu Leu Tyr Arg Tyr Ile Asn Lys Ala Phe Pro Gly Lys
   290 295 300
   Leu Arg Arg Leu Met Lys Lys Met His Cys Gly Arg Arg His Cys Ser
   305 310 315 320
   Pro Asp Pro Ser Gly Ile Gln Gln Val Met Ala Gln Ala Glx
   325 330
<210> 7
   <211> 7321
   <212> DNA
   <213> Mus musculus
<400> 7
   ccccgtcact ggacaaactc aggcatcccc tggcaggcgc agaatatagc tctgtggaga 60
   ccaccagacg atttctcccc agcctcccac tggtttatga tccagtttct caggaaggaa 120
   attaccagat ggcctggcta actggaaaga cagcctagcc caggtcagag ggtggaaacc 180
   aaatcagtgc tcactggcta caggaagtcc atcttctcca gccgcaaggg gaacaggaag 240
   gaagatgcag ggagacagga accctgggaa gcctgtgagt acatccacac ccacatgcta 300
   tgggggccag cctccccaat tcccttctgt gggtagggag aacagttagc tgagaccgct 360
   ggactcgctg ggatactact gttaggtgcc cacagcctca acagggggaa ccccactgga 420
   gggtacccca agtggaactg actggattga gggtccccaa ggccagagct ctacagccaa 480
   gcagagactt gctatggttc tggggcctcg cctgctgctc ccagctgtca ttaggccacc 540
   cccaaccgtt cctccccaag ctgcagccca caccttaagg tggccacagg gacattccca 600
   gggcagagaa gctgggacgg atgctctagt gacagtgtca gacctatgcc tctgacgtgg 660
   ttggtgcagc ctacaaagcc gctctgctct tgggctctcc ccgctgtgcc tgctgggagg 720
   ggcatctcca agagcaacac catagcaagc cttgaagaat atcacttcta caaagtaaat 780
   gatagcaagt atgattctgc cttttttatt tttttatctg aaataaaagt tctgatgagt 840
   aagaaagttt tgaacagcag ttacccgcta gctgctagac aaagcttatc aggtgttcca 900
   cactcacagg ctggcgagaa gcacctctga ggtacatgta gcctggataa acagcacagc 960
   acagctctca gacccaggcc atggcaggca gcacatgaac atggagggaa ttctacccag 1020
   acagacacta cgttgttcca catgacgctt tccctaaagc ccagctgctc gcccagtgcg 1080
   ggccctgagc cctacctcct gccccacttc tcactgcagg ccattagcag tccgggcagc 1140
   tgccgtgccc accaggctgc atgggggtca tggagggcaa gcagtcctgc caggccacag 1200
   cactgttgcc aagtagtagc tctcccctgt tcttgtggcc ccagacgggt cacatgcacg 1260
   cacatctgga aatgcacgtg tgggaggctt tggaccccct gccccagctc tggtgttttc 1320
   aattctaagc ctgctctgtg ccaccactgt gccttcaaag agccttgtgt gtccctgggc 1380
   ctgcagtgaa agcagccagc atctctatgg cggagtctca gagaggtcca actattttgt 1440
   ccttagttct acagacaaat gtctgtttag ttcacttcct caagggaaaa tggaactcag 1500
   gacagaacta aactgctccg ggtcccagga cctgagctgc ctgtacccaa cctgcaggcc 1560
   ctcccttgcc caggggctgg atgctcttca gggcagatac agaacatccc tccacccacc 1620
   cacccccagc acagtgcaga ggatgtgagc acacttgatc tggagggcag actgtccaag 1680
   tgccgcctct cctcccgaaa ccagctgtaa acacagatag ggaggggcct ccgtgcctgc 1740
   caagggaggc agcaactctg tgagtaattg actcaaggca aacaagtgag gcaggagttg 1800
   tgtgtggtgc tcacgagcat cgctggctgg ggctcctgag acacaggccc tggcccggca 1860
   tgctgcctgc tgctggcctc tggagcccac agcacagagg aaggagggac ttgtaataaa 1920
   ggtaggagcc tgatggctgt cactcactgc tactggctta gtggggcaga ggatgaggag 1980
   gctagaactg gtgacttgcc accaatgtgg gtcacataga catgagcgag aggaacagag 2040
   cagaatcctt ggacagagtg agttcctgga cagatctatt caatatatat ctacaatgca 2100
   aggtctggag ggcatagtcc ctgtcatcag acttgccata aagcctgagt ccataatgcc 2160
   agccaaggcc aggtcagcag cagtgggggg gggggggtag tgaccaggag aagacagagc 2220
   tcaactaata cccatccaca gtgcccacag agtacccgct gtactggctg gttttgtgtc 2280
   aacttgacac aagctggagt tatcacagag aaaggagctt caggtgagga aatgcctcca 2340
   tgagatccag ctgtggggca ttttctcaat tagggatcaa gggggaaagg ccccttgtgg 2400
   gtgggaccat ccctgggctg gtgagtcttg ggttctataa gagagcaggc tgagcaagcc 2460
   agtaaagaac atccctccat ggcctctgca tcagctcctg cttcctgacc tgcttgagtt 2520
   ccagtcctga cttcctttag tgatgaacag cagtgtggaa gtgtaaactg aataaaccct 2580
   ttcctcccca acttgcttct ttgtcatgat gttggtgcag gaatagaaac cctaactaag 2640
   acaacctacc cttccctgag gactgtgaca ctgtgatctg atgggtaagg ctccaataac 2700
   gtgtggcacc agtagccacc aaacccaggg gtcacatatc ctttatctcg ggccttctgt 2760
   gctaagtgct aggggaggtg ccacagaaca gaagggaaga gaagagcggt cccaggggag 2820
   gtgaccagaa atgatagaca ccacaaagag acacacttca ggagttggtg tacaccgacc 2880
   ctccaagggc cagtttctag ccccccatga agcaggcagg ggattgtctg gtggaggata 2940
   tagcaagcac ttgtccttaa aggaggtcag ggccacttag tactaggcag ggccccactc 3000
   cttgccttcc cctagctgct gggagaacag gtgaggactg cagggtcttt actcagtggc 3060
   ctcccatgcc tgcatctgaa tgcatcccag catcacctgc cgagcggatc cctgtgtgcc 3120
   tgacctgggg aggaagacag gctgtgtaca gacacacagg ctgtggagca ggcctcccct 3180
   tcccccagac ctcgctgcca gtgtgcatct gtgctggcag aagggccctc cactcactgc 3240
   tgtgacccac agcccacact cctgcagtcg gctgtggaga gcaagtgggg gacagccctt 3300
   agtcccagcg gggacccaag gcagcaggac cagtgccaac accatcagcc tggcttcctc 3360
   gctttgtccc tgcactctgg gaaacaggaa tgttcgcttc cagtgtgtct gctcggggtg 3420
   ctgtttctct tttgattttg tgcctggagg tctagttggg ccaaggtgta gtttgaatgt 3480
   aaagttcccc actaaagaac tgggtgtccc cgaaggacag gtgaagagga attctctata 3540
   gacccacttg ccaaactcgc actcacttaa catcaagact gactggacag actgcaacat 3600
   gggctctgtg ctgccccgtg ggtcagtgac catgggggag gaaaggaatg cccgggctca 3660
   cccaggcctc ccacgtctcc cagcattgct ggatctaggt gcccagcaac ctagcaagct 3720
   tcaagataac tacaaagggc ctgcccatct gagggaaatt atgtgggcac aatgcagcca 3780
   gcatccatgt tcagtcccag gccttgtcag cctgggaggt gcctgtgcct cctgcctcct 3840
   gcctccacac acctcccggg cagctcttca ggccccgctc tcagggtacg gtgcaggggg 3900
   ctacaggtgt gcaacaccca ggcaggtgtg tctccacatt accagatgac aggctatact 3960
   taggtcctct acacaagaaa gagggggcaa ggaggcaggc agccttgcac tgccaagtgg 4020
   tttgaggttc tgtgggttct gtccccaaag agtcccctaa cccgtgccaa aaggtagcag 4080
   cagctctgct cctggtggag tagtggcaca ggaaggcaca caaaagcctc tctggtgggt 4140
   cacctgggat tttggtttcc ttcataaaga gtccaggagt ctcaccaagg cgatgaatgg 4200
   ctccagccgc ctgacccgga cacacacaca cgctggcaga tgccggcaca ggacagtggt 4260
   gactaactct gtccctctct ctcaacagct gcgaagctgg ggtgagctgc catgtggagc 4320
   tgtggcccac tcaacagcac agcgtgggct gaggagccgc tgtgccggaa cctgcgcctg 4380
   gggctgtggg tcctctcgct gctctacctg ggggcagggg tccctgtgag cttaggctat 4440
   aatgctcttc tggtgctggc caacctggcc agcaagaaca ccatgaccat gcccgacgtg 4500
   tacttcgtga acatggccgt ggcggggctg gtgctcacgg cactggcacc tgcgtacctg 4560
   ttgggccctg cccactccag gtgggccctg tggagcctca gcagtgaggc ccatgtgaca 4620
   ctgctcatcc tgttcaacgt ggcttccctg gtgaccatgt actccactgc actgctgagc 4680
   cttgactact acatcgagcg tgccctgcca cgcacctaca tggccagtgt gtacaacacc 4740
   cggcacgtgt gtggcttcgt ctggggaggg gcggtgctca ccagcttctc ctccctgctc 4800
   ttctacatct gcagtcacgt gtcttctaga atcgctgagt gtgcccggat gcagaacacg 4860
   gaggcagccg atgctatcct tgtgctcatc ggctacgtgg tgccaggtct ggctgtgttg 4920
   tatgccctgg cactcatctc gagaatcggg aaggaagaca cacccctgga ccaggacacc 4980
   agcaggctgg acccctcggt gcacaggctg ctggtggcca ccgtgtgcac tcagtttggc 5040
   ctctggacac cttactactt gagcctgggg cacacagtgc tgacgtcacg ggggaggacc 5100
   gtggaggggc attatctggg catcctacag gttgctaagg acctggccaa gttcctggcc 5160
   ttctcaagca gttctgtgac accactgctc taccgttaca tcaacaaagc cttccccggc 5220
   aagctccggc ggctgatgaa gaagatgcac tgcgggcgcc gccactgctc ccccgacccc 5280
   tcggggatac agcaggtgat ggcacaggcg tagctaaccc tccctgggct gaccacgagg 5340
   aacactaaaa ctcaactgga cacatagcac tttgttcccc aggcacatgg agcttccagc 5400
   ccaggagaca cgctgaagcc agagatgcac agcggggcat ttctcttgat gtttcctgtt 5460
   ttcccctaaa aggccaacct tggtcttatg ctgcatggtg gaaagcagag gtgccctgtg 5520
   tgaacaggct gcttctgact aaccttccca ccttctccct caaggtgtgt gcctttctcc 5580
   cagggacatc ttcaaggtcc atgtcctcac aagcaggctg accttcggcc tccctggtat 5640
   ttgggctttc taaatgaagc gatgaaatct aaagccagta tttatacttc atattgacat 5700
   gatacttgat tctcctatta ttattttttt aaaaatagaa gtgttagaaa gatacctagt 5760
   attaagaaga aggcacttca ggggaatgtg gctgtcgctg ataccagtgt tagagcttga 5820
   ggacaggaca gtgggcctgt caggcctgca gcagctacac agccattgct gacagtgaca 5880
   gctatggagc actggccctc tcctctactg agagaggatc tttccagtgc ttcaacatat 5940
   gcatatacat aaatggaaaa aaaagaagtg tgttctctta gtgccaaagg ccttctggag 6000
   acctggatat ggtagagggt tagccaggca tagaaggaaa aggcaggatg gtatgagacc 6060
   acggctccaa gatgaccagg gactggcatc agggaaaggg ccctgcttgg ccagtttcac 6120
   acagctgtgg gtggcacatg aggaggctca cctcccacag gtgcctggat ccaggcctca 6180
   cccacgtgct tctggggtct tttaccatcc tcatgtttgg taattatcta ctctaatgtg 6240
   ggcatgaaaa gaattcccac cacccgaatt ctctcggcta catggcaagc ccacaagtcc 6300
   ctgcccccag ttctaaagct ctccatgtga ccatctcacc cctgtctcta cagaaggcgg 6360
   gtgtgtgacc cacattcagt ggccttcttg catggcccca cagctggtcc tggctgtctg 6420
   ggaactgctg ctctgagtca gtaactttag tgttatctat tctatgcaaa ctccatctcc 6480
   atggacggtg acggctccag ttaatctgcc ttaaggagaa acacgaggcc cgcagcccta 6540
   gctgtgacct actgggcatg ctctgtgggt ctcagttcat gaataaacca aagggctggg 6600
   ttagacaggc atcttcagag ttgcttacgg ctgtagaatt ttgttttgag gactttaaat 6660
   tggcaaacct gactttccct acccccgccc caggtgcaca gctgagtgaa ggctctgaaa 6720
   agctaatggc ttccaggctt cccaccctga tagccacaat gaacacctta acgcttgctg 6780
   aggaaacaag aaccctcacc atgaaggttc tagctagacc tggcctgcag gccacggtaa 6840
   ttctcctatg agagcaagcc cagggaccaa ggtcacatca aacaaaaggc aacctatgct 6900
   ctgggggtca tagaggaaac aggcttcagg tgggcagggc tgtttgtgat gttggcgccc 6960
   cagtttaaca gtctttgccc tggggaccgt tttataccac ttactccagg cagaggtcca 7020
   gagaggggca gacccccagg tccaagtggg gttgtacgcc ctagaaccct ccaccccagt 7080
   cacccccaag catgatagag agaggcccaa accatgttcc tttgttgcac tttttcagtc 7140
   acattaggta caaactggcg aggttctagg ggacccttga ggggttggaa gccaggacca 7200
   ggaatgtgtg actgccatca attgtatagc caatcactgc ctacctgggt cttccttgtg 7260
   gagccgcaca tggccagggt agtggccatc tgcagtgctg acccctttgc tgtagagacc 7320
   c 7321
<210> 8
   <211> 27
   <212> DNA
   <213> Artificial
<220>
   <223> Oligonucleotide Primer (See Table 1)
<400> 8
   ccccgtcact ggacaaactc aggcatc 27
<210> 9
   <211> 27
   <212> DNA
   <213> Artificial
<220>
   <223> Oligonucleotide Primer (See Table 1)
<400> 9
   ctgttcccct tgcggctgga gaagatg 27
<210> 10
   <211> 37
   <212> DNA
   <213> Artificial
<220>
   <223> Oligonucleotide Primer (See Table 1)
<400> 10
   tttgcggccg ccttctgtgg gtagggagaa cagttag 37
<210> 11
   <211> 36
   <212> DNA
   <213> Artificial
<220>
   <223> Oligonucleotide Primer (See Table 1)
<400> 11
   tttactagtc tgttgagtgg gccacagctc cacatg 36
<210> 12
   <211> 37
   <212> DNA
   <213> Artificial
<220>
   <223> Oligonucleotide Primer (See Table 1)
<400> 12
   aaaggcgcgc ctctaccgtt acatcaacaa agccttc 37
<210> 13
   <211> 38
   <212> DNA
   <213> Artificial
<220>
   <223> Oligonucleotide Primer (See Table 1)
<400> 13
   aaaggccggc cataaaacgg tccccagggc aaagactg 38
<210> 14
   <211> 27
   <212> DNA
   <213> Artificial
<220>
   <223> Oligonucleotide Primer (See Table 1)
<400> 14
   tctctggacc tctgcctgga gtaagtg 27
<210> 15
   <211> 27
   <212> DNA
   <213> Artificial
<220>
   <223> Oligonucleotide Primer (See Table 1)
<400> 15
   tccaagtggg gttgtacgcc ctagaac 27
<210> 16
   <211> 27
   <212> DNA
   <213> Artificial
<220>
   <223> Oligonucleotide Primer (See Table 1)
<400> 16
   gggtctctac agcaaagggg tcagcac 27
<210> 17
   <211> 27
   <212> DNA
   <213> Artificial
<220>
   <223> Oligonucleotide Primer (See Table 1)
<400> 17
   ctcatctcga gaatcgggaa ggaagac 27
<210> 18
   <211> 27
   <212> DNA
   <213> Artificial
<220>
   <223> Oligonucleotide Primer (See Table 1)
<400> 18
   gaaggctttg ttgatgtaac ggtagag 27
<210> 19
   <211> 27
   <212> DNA
   <213> Artificial
<220>
   <223> Oligonucleotide Primer (See Table 1)
<400> 19
   cagccgaact gttcgccagg ctcaagg 27
<210> 20
   <211> 27
   <212> DNA
   <213> Artificial
<220>
   <223> Oligonucleotide Primer (See Table 1)
<400> 20
   aacgcacggg tgttgggtcg tttgttc 27

## Description

### Field of the Invention

This invention relates to G-protein coupled receptor (GPCR) polypeptides and nucleic acids, hereinafter referred to as "GPR146 GPCR", which is a member of the family of GPCRs. The invention also relates to inhibiting or activating the action of such nucleic acids and polypeptides, in particular to provide candidates for the therapy of disorders of lipid metabolism.

### Background to the Invention

Many medically significant biological processes are mediated by proteins participating in signal transduction pathways that involve G-proteins and/or second messengers, for example, cAMP (Lefkowitz, Nature, 1991, 351: 353-354). These proteins are referred to as "proteins participating in pathways with G-proteins" or "PPG proteins". Examples of PPG proteins include the GPCRs, such as those for adrenergic agents and dopamine (Kobilka, B. K., et al., Proc. Natl Acad. Sci., USA, 1987, 84: 46-50; Kobilka B. K., et al., Science, 1987, 238: 650-656; Bunzow, J. R., et al., Nature, 1988, 336: 783-787), G-proteins themselves, effector proteins, for example, phospholipase C, adenyl cyclase, and phosphodiesterase, and actuator proteins, for example, protein kinase A and protein kinase C (Simon, M. I., et al., Science, 1991, 252: 802-8).

In one form of signal transduction, hormone-binding activates adenylate cyclase inside the cell. A G-protein connects the hormone receptor to adenylate cyclase. G-protein exchanges GTP for bound GDP when activated by a hormone receptor. The GTP-bound form then binds to and activates adenylate cyclase. Hydrolysis of GTP to GDP, catalysed by the G-protein itself, returns the G-protein to its basal, inactive form. Thus, the G-protein serves a dual role, as an intermediate that relays the signal from receptor to effector, and as a clock that controls the duration of the signal.

The membrane protein gene superfamily of GPCRs is characterised by seven putative transmembrane domains, which are believed to represent transmembrane α-helices connected by extracellular or cytoplasmic loops. GPCRs (also known as 7TM receptors) include a wide range of biologically active receptors, such as hormone, viral, growth factor and neuroreceptors. The seven conserved hydrophobic stretches of about 20 to 30 amino acids, connect at least eight divergent hydrophilic loops. The 7 transmembrane regions are designated as TM1 to TM7, respectively. TM3 has been implicated in signal transduction. The GPCR family includes dopamine receptors which bind to neuroleptic drugs used for treating psychotic and neurological disorders. Other members of this family include, but are not limited to, calcitonin, adrenergic, endothelin, cAMP, adenosine, muscarinic, acetylcholine, serotonin, histamine, thrombin, kinin, follicle stimulating hormone, opsins, endothelial differentiation gene-1, rhodopsins, odorant, and cytomegalovirus receptors.

Most GPCRs have single conserved cysteine residues in each of the first two extracellular loops, which form disulphide bonds that are believed to stabilise protein structure. Phosphorylation and lipidation (pamitylation or farnesylation) of cysteine residues can influence signal transduction of some GPCRs. Most GPCRs contain potential phosphorylation sites within the third cytoplasmic loop and/or the carboxy terminus. For several GPCRs, such as the β -adrenoreceptor, phosphorylation by protein kinase A and/or specific receptor kinases mediates receptor desensitization. For some receptors, the ligand binding sites of GPCRs are believed to comprise hydrophilic sockets formed by several GPCR transmembrane domains, the sockets being surrounded by hydrophobic residues of the GPCRs. The hydrophilic side of each G-protein coupled receptor transmembrane helix is thought to face inward and form a polar ligand binding site. TM3 has been implicated in several G-protein coupled receptors as having a ligand binding site, such as the TM3 aspartate residue. TM5 serines, a TM6 asparagine and TM6 or TM7 phenylalanines or tyrosines are also implicated in ligand binding.

GPCRs can be intracellularly coupled by heterotrimeric G-proteins to various intracellular enzymes, ion channels and transporters (see, Johnson et al., Endoc. Rev., 1989, 10: 317-331). Different G-protein α-subunits preferentially stimulate particular effectors to modulate various biological functions in a cell. Phosphorylation of cytoplasmic GPCR residues is an important mechanism for the regulation of G-protein coupling of some GPCRs. GPCRs are found in numerous sites within a mammalian host. Nearly 350 therapeutic agents targeting GPCRs have been successfully introduced onto the market.

GPCRs have an established, proven history as therapeutic targets. Clearly there is a need for identification and characterization of further receptors that can play a role in preventing, ameliorating or correcting disorders, dysfunctions and diseases.

Dyslipidaemias are diseases that involve a disorder of lipid metabolism, in particular lipoprotein metabolism, including lipoprotein excess or deficiency, resulting in an altered level of lipids in the blood. A well-known lipid that is commonly elevated in the blood, in particular in Western society, is cholesterol or cholesterol esters.

Coronary heart disease is the leading cause of death in the United States as well as in most developed countries. Atherosclerosis is the underlying disease damaging the endothelium lining the arteries as well as the intima beneath. Besides coronary heart disease atherosclerosis causes similar vascular changes in other arteries, leading to a variety of diseases such as stroke, transient reversible ischemic neurological deficits, aneurysms and endangitis obliterans (intermittent claudicatio).

The molecular and cellular events leading to the development of atherosclerosis have been elucidated by several decades of intense scientific work. Low density lipoproteins (LDL) and other apo-B containing lipoproteins circulating in the blood are deposited in the subendothelial connective tissue of the intima where they undergo several modifications such as glycosylation or oxidation. These modified lipoproteins are taken up by macrophages resulting in accumulation of cholesterol and cholesterylesters in these cells. This causes a morphological shift towards a foam cell which induces proliferation of smooth muscle cells and fibroblasts, and release of cytokines promoting a local inflammatory response. The centre of the atheromatous plaque contains necrotic material, surrounded by foam cells and proliferated fibroblasts, smooth muscle cells, altered extra-cellular matrix and immune cells. This plaque is covered by endothelium which is also altered in its functional state. The plaque causes ultimately a narrowing of the arteries (stenosis) reducing the blood flow. The plaque and the endothelium covering it may rupture at some stage and cause a thrombotic event leading to the complete closure of the artery. Alternatively, the plaque is a locus minoris resistentiae in the vessel wall and this might lead to rupture of the wall with consequent massive bleeding.

The complex interplay between several risk factors contributes to the development of atherosclerosis. One separate factor is smoking, whereas others are summarised under the term "metabolic syndrome" (synonymous with "syndrome X"). The metabolic syndrome is a cluster of metabolic abnormalities including abdominal obesity, glucose intolerance, hypertension, a pro-thrombotic state and dyslipidaemia, specifically hypercholesterolemia. Some definitions include also a pro-inflammatory state which is usually demonstrated by the presence of pro-inflammatory cytokines such as TNF-alpha or II-6 in the serum. Despite some differences in the exact definition of the metabolic syndrome it is usually diagnosed if at least 3 of the features mentioned are present. A large proportion of patients with metabolic syndrome progresses to full blown atherosclerosis and/or type 2 diabetes. (Daskalopoulou et al., 2006: Definitions of metabolic syndrome: Where are we now? Curr Vasc Pharmacol 4, 185-197).

The correlation between dyslipidemia, specifically hypercholesterolemia, and atherosclerosis is well established and has triggered a major effort to diagnose, educate and treat persons at risk. This campaign has been addressing the individual risk factors of the metabolic syndrome and smoking by a combination of public health education efforts and pharmacological intervention with some success (Carroll et al., 2005: Trends in serum lipids and lipoproteins of adults, 1960-2002. Jama 294, 1773-1781.; Gregg et al., 2005: Secular trends in cardiovascular disease risk factors according to body mass index in US adults. Jama 293, 1868-1874.). Nevertheless, cardiovascular disease is still the leading cause of death in the developed world, and the risk factors are present in the overwhelming majority of the patients (Ford et al., 2002: Prevalence of the metabolic syndrome among US adults: findings from the third National Health and Nutrition Examination Survey. Jama 287, 356-359.; Khot et al., 2003: Prevalence of conventional risk factors in patients with coronary heart disease. Jama 290, 898-904.). Recent clinical studies have demonstrated that further reduction of LDL-cholesterol exceeding the current suggestions is beneficial as measured by clinical outcomes (Fitchett et al., 2006 Lower is better: implications of the Treating to New Targets (TNT) study for Canadian patients. Can J Cardiol 22, 835-839.). As this aggressive treatment requires significantly higher doses of the available drugs, mainly statins, ezetimibe, fibrates and nicotinic acid, the side effects are expected to become more prominent. A possible solution, at least to some extent, might be combinations of available drugs, but new strategies for cholesterol reduction are clearly necessary (Beaven and Tontonoz, 2006: Nuclear receptors in lipid metabolism: targeting the heart of dyslipidemia. Annu Rev Med 57, 313-329.).

There is clearly a massive need for new, effective therapies and diagnostic assays for dyslipidaemias such as atherosclerosis and CHD.

### Summary of the Invention

The present invention is based on the surprising realisation that GPR146 is implicated in disorders of lipid metabolism and is therefore a useful therapeutic and diagnostic target. The inventors have found that, surprisingly, animals lacking a functional GPR146 gene demonstrate a significant reduction in serum cholesterol levels compared to wild type animals. Therefore, the GPR146 receptor is implicated in lipid metabolism and is a therapeutic and diagnostic target for disorders of lipid metabolism.

According to a first aspect, the present invention provides an *in vitro* or *ex vivo* method for identifying an agent suitable for therapy of a disorder of lipid metabolism selected from atheromatous disease, atherosclerosis, coronary heart disease, gall bladder disease, impaired glucose tolerance, metabolic syndrome X, restenosis, septic shock, stroke, angina pectoris, transient ischemic attacks (TIAs) or peripheral artery disease (PAD) comprises the step of determining whether a candidate affects the serum cholesterol controlling activity of GPR146, wherein GPR146 comprises or encodes a polypeptide identified herein as SEQ ID No. 4 or SEQ ID No. 6, or a sequence with at least 70% sequence identity thereto.

According to a second aspect, the present invention provides, a non-human transgenic animal having a functionally disrupted endogenous GPR146 gene, wherein the GPR146 gene is as defined in the first aspect of the invention.

According to a third aspect, the present invention provides a method for determining the presence or susceptibility to a disorder.of lipid metabolism defined in the first aspect of the invention in an individual comprising the steps of (i) determining the expression level of GPR146 gene in a sample isolated from the patient and (ii) determining, compared to a control, whether the individual from which the sample was isolated has or is susceptible to a disorder of lipid metabolism defined in the first aspect of the invention, wherein the GPR146 gene is as defined in the first aspect of the invention.

According fourth aspect, the present invention provides for the use of an exogenous GPR146 polynucleotide as defined in the first aspect of the invention in the manufacture of a medicament for the treatment or prevention of a disorder of lipid metabolism defined in the first aspect of the invention.

### Description of Drawings

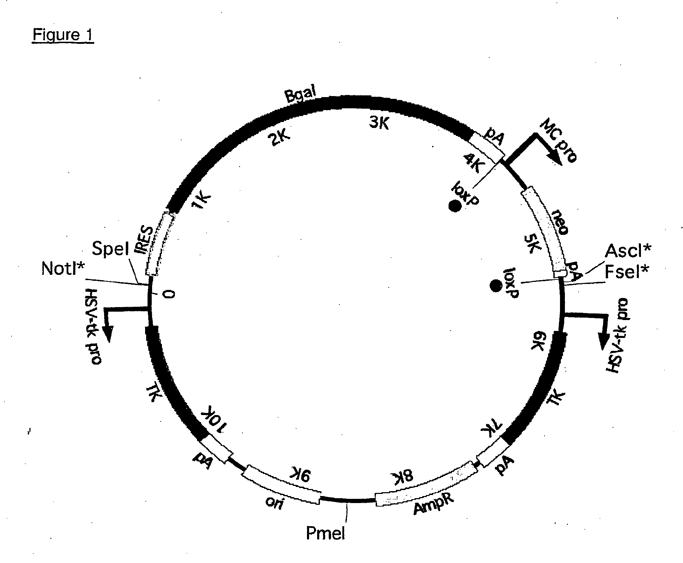

Figure 1 is a diagram showing the knockout vector;
Figure 2 illustrates the decrease in serum cholesterol seen in GPR146 knockout mice of different ages and sexes when compared to wild type, the reduction in cholesterol levels in knockout mice is statistically significant in both males and females (Student's T-test p<0.05); and
Figure 3 shows the genomic sequence of mouse GPR146 (also inlcuded as Seq ID No.7), indicating the location of the primers used in the targeting strategy.

### Sequence Listings

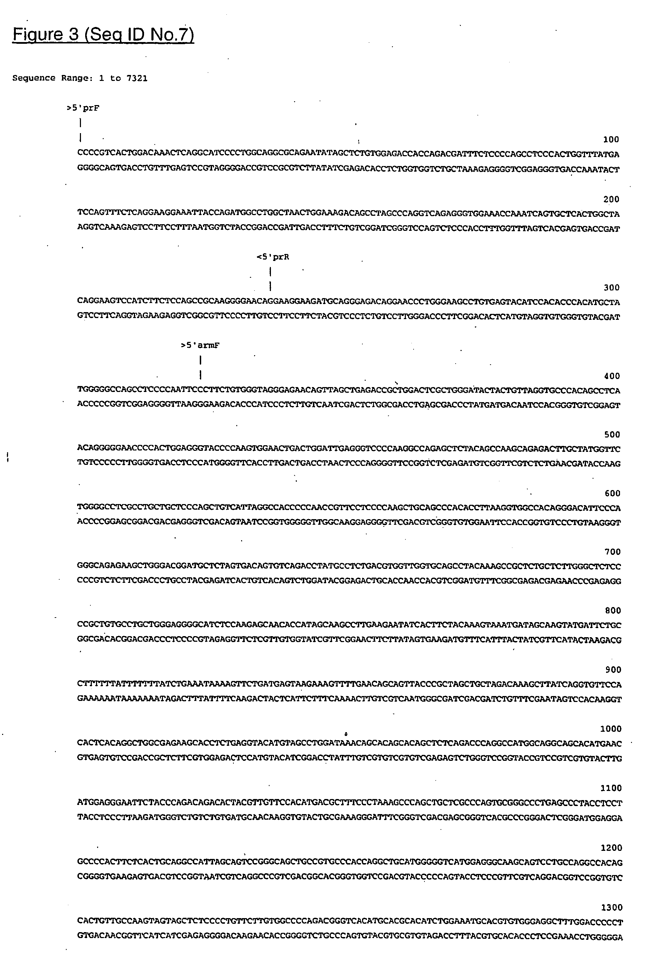

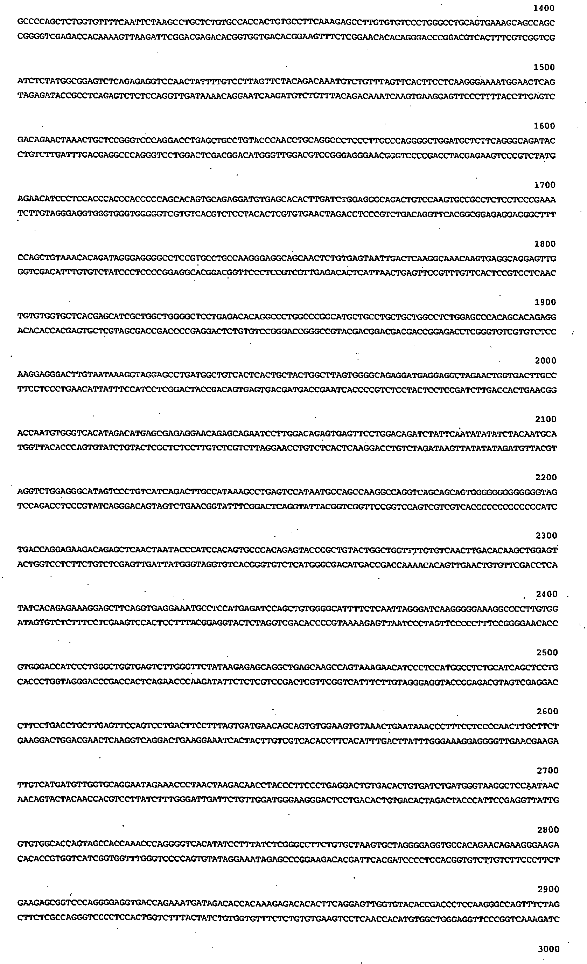

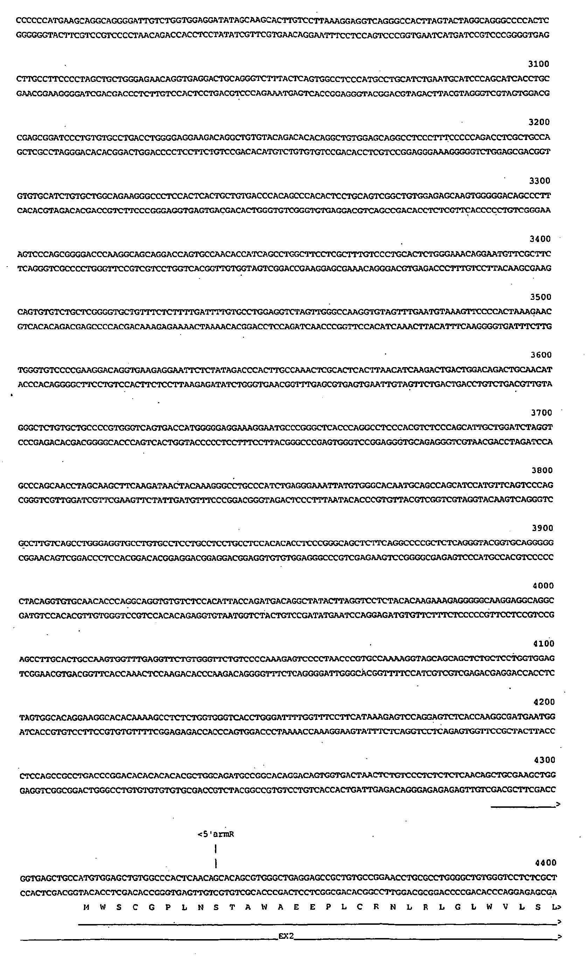

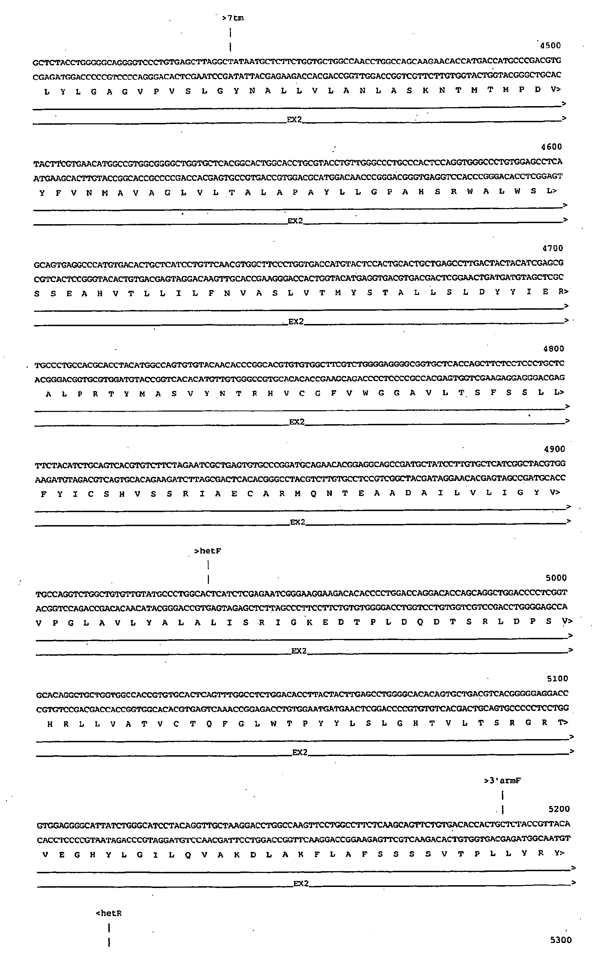

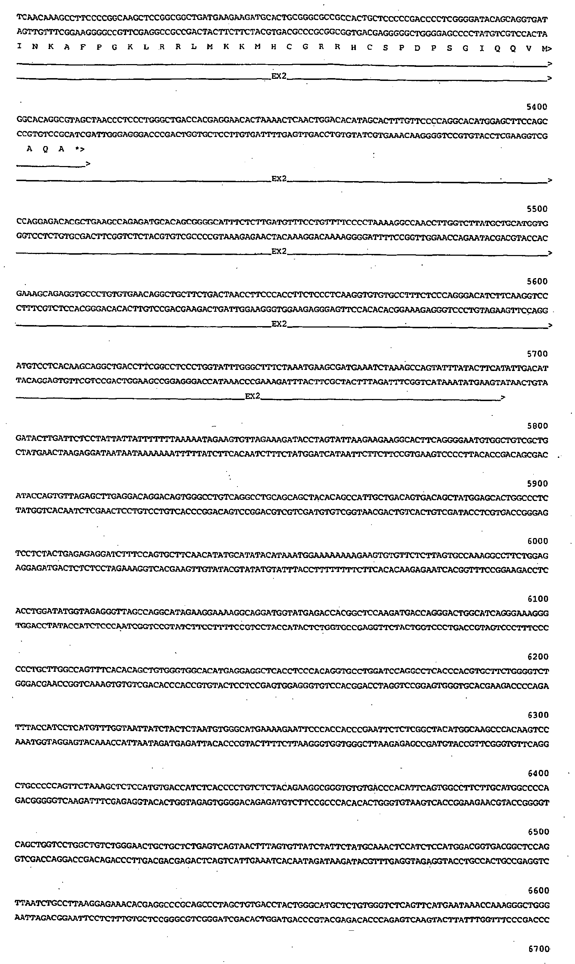

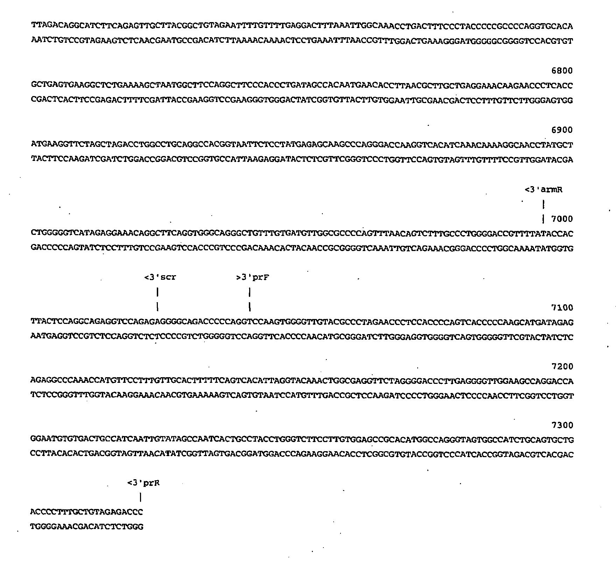

SEQ ID NO: 1 and SEQ ID NO: 2 show two cDNA sequences of human GPR146. SEQ ID NO: 3 shows an open reading frame derived from both SEQ ID NO: 1 and SEQ ID NO:2. SEQ ID NO: 4 shows the amino acid sequence of human GPR146. SEQ ID NO: 5 shows the open reading frame of a cDNA for Mouse GPR146. SEQ ID NO: 6 shows the amino acid sequence of Mouse GPR146. SEQ ID NO: 7 (and Figure 3) shows the genomic sequence of Mouse GPR146 from the 5' arm (5' prF) to the 3' arm (3' prR). SEQ ID NOs: 8 to 20 show the nucleotide sequence of Mouse GPR146 primers used in construction and analysis of the targeting vector and agents containing the targeted locus.

### Detailed Description of the Invention

### GPR146 GPCR

The invention relates to a novel GPCR, which is a rhodopsin type of 7 transmembrane receptor which is referred to as GPR146 GPCR, as well as homologues, variants or derivatives thereof.

GPR146 is structurally related to other proteins of the G-protein coupled receptor family, as shown by the results of sequencing the amplified cDNA products encoding human GPR146. The cDNA sequences of SEQ ID NO: 1 and SEQ ID NO:2 contain an open reading flame (SEQ ID NO: 3) encoding a polypeptide shown in SEQ ID NO: 4. Human GPR146 is found to map to chromosome 7p22.3.

### Identities and Similarities to GPR146

Analysis of the GPR146 polypeptide (SEQ ID NO: 4) using the HMM structural prediction software of pfam (http://www.sanger.ac.uk/Software/Pfam/search.shtml), together with a 7TM prediction analysis, confirms that GPR146 peptide is a GPCR of the 7TM-1 structural class.

The mouse homologue of the human GPR146 GPCR has been cloned, and its nucleic acid sequence and amino acid sequence are shown as SEQ ID NO: 5 and SEQ ID NO: 6 respectively. The mouse GPR146 GPCR cDNA of SEQ ID NO: 5 shows a high degree of identity with the human GPR146 GPCR (SEQ ID NO: 3) sequence, while the amino acid sequence (SEQ ID NO: 6) of mouse GPR146 GPCR shows a high degree of identity and similarity with human GPR146 GPCR (SEQ ID NO: 4).

Human and mouse GPR146 GPCR are therefore members of a large family of GPCRs.

### GPR146 GPCR-Associated Diseases

According to the methods and compositions described here, GPR146 GPCR is useful for treating and diagnosing a range of disorders of lipid metabolism, in particular lipoprotein metabolism, including lipoprotein excess or deficiency, resulting in an altered level of lipids in the blood. Lipoproteins that can be increased or decreased in a dyslipidaemia are chylomicrons, Low Density Lipoproteins (LDL), Intermediate Density Lipoprotein (IDL), High Density Lipoproteins (HDL) and Very High Density Lipoproteins (VLDL). The skilled person will recognise that lipids are transported in the blood as lipoproteins.

In a preferred embodiment the disorder of lipid metabolism involves an increased level of lipid or lipoprotein in the blood, preferably the serum, compared to a non-diseased control level. In an alternative embodiment, the disorder of lipid metabolism involves a decreased level of lipid or lipoprotein in the blood, preferably the serum, compared to a non-diseased control level.

Without wishing to be bound by theory, the observation of reduced cholesterol levels in GPR146 deficient mice may be due to reduced endogenous synthesis or increased excretion with the bile. Both mechanisms are useful to treat a hypercholesterolemia as shown by the efficacy of statins (reduction of endogenous synthesis) or resins that increase excretion of cholesterol and the products of its metabolism, bile acids. Using the mouse as a model organism we have shown that a reduced or absent function of GPR146 causes a hypocholesterolemia. This indicates that the physiological function of GPR146 is the modulation of the cholesterol metabolism. In analogy, an antagonist inhibiting the function of GPR146 in a hypercholesterolemic patient would reduce the pathologically elevated cholesterol level and thus reduce the further development of atherosclerosis and also its manifestations in different organs such as coronary heart disease, angina pectoris, stroke, transient ischemic attacks (TIA), peripheral artery disease, stenosis or aneurysms of the carotid or fermoral artery, or of the aorta.

In addition to these beneficial effects on diseases of lipid metabolism, including atherosclerosis, a reduction in serum cholesterol is usually associated with changes in the plasma membrane composition. Receptors for several hormones or local signalling factors have been shown to be embedded in specialised cholesterol-enriched plasma membrane domains known as lipid rafts. The raft composition is essential for the efficacy of signal transduction. This mechanism has been shown to modulate the insulin receptor in skeletal muscle, adipocytes and the liver, and a reduction in serum cholesterol levels is predicted to improve the insulin resistance (i.e. increase the sensitivity of the cells to insulin). This effect would be synergistic with the reduction in serum cholesterol by improving 2 of the risk factors of the metabolic syndrome. Furthermore, reduced cholesterol synthesis or absorption from the gastrointestinal tract results in less cholesterol excretion. The major pathway of cholesterol excretion is via the bile, either as cholesterol or its metabolites, the bile acids. Reduced secretion of bile acids and cholesterol reduced the lithogenicity of the bile fluid, thus reducing gall bladder stones.

According to a preferred embodiment of the invention, an exogenous GPR146 polynucleotide may be used in the manufacture of a medicament for the treatment or prevention of a disorder of lipid metabolism selected from atheromatous disease, atherosclerosis, coronary heart disease, gall bladder disease, impaired glucose tolerance, insulin resistance, metabolic syndrome, restenosis, stroke, angina pectoris, transient ischemic attacks (TIAs) and peripheral artery disease (PAD).

Known diseases which have been determined as being linked to the same locus, chromosomal band, region, arm or chromosome as the chromosomal location of Joseph GPCR (i.e., chromosome 7p22.3) include Familial Hyperaldosteronism Type II.

As noted above, an exogenous GP146 polynucleotide may be used to diagnose, prevent or treat any of these specific diseases using any of the methods and compositions described here.

### Methods Employed

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

### GPR146 GPCR Polypeptides

As used here, the term "GPR146 GPCR polypeptide" refers to a polypeptide comprising the amino acid sequence shown in SEQ ID No. 6 or more preferably SEQ ID NO: 4, or a homologue, variant or derivative thereof. "Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids.

"Polypeptides" include amino acid sequences modified either by natural processes, such as post-translational processing, or by chemical modification techniques which are well known in the art. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. A given polypeptide may contain many types of modifications.

Polypeptides may be branched as a result of ubiquitination, and they may be cyclic, with or without branching. Cyclic, branched and branched cyclic polypeptides may result from post-translation natural processes or may be made by synthetic methods. Many protein modifications are known, see, for instance, Proteins - Structure and Molecular Properties, 2nd Ed., T. E. Creighton, W. H. Freeman and Company, New York, 1993 and Wold, F., Posttranslational Protein Modifications: Perspectives and Prospects, pgs. 1-12 in Posttranslational Covalent Modification of Proteins, B. C. Johnson, Ed., Academic Press New York, 1983; Seifter et al., "Analysis for protein modifications and nonprotein cofactors", Meth Enzymol (1990) 182:626-646 and Rattan et aL, "Protein Synthesis: Posttranslational Modifications and Aging", Ann NYAcad Sci (1992) 663:48-62.

The terms "variant", "homologue", "derivative" or "fragment" as used herein include any substitution, variation, modification, replacement, deletion or addition of one (or more) amino acid from or to a sequence. The variant may have a deletion, insertion or substitution variation that produces a silent change and a functionally equivalent polypeptide. Deliberate amino acid substitutions may be made on the basis of similar physio-chemical properties such as size, charge and hydrophobicity.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I LV |
| | Polar - uncharged | C S T M |
| | | NQ |
| | Polar - charged | D E |
| | | KR |
| AROMATIC | | H F W Y |

Unless the context admits otherwise, references to "GPR146" and "GPR146 GPCR" include references to such variants, homologues, derivatives and fragments of GPR146.

Preferably, as applied to GPR146, the amino acid sequence has GPCR activity, more preferably having at least the same activity of the GPR146 GPCR shown as SEQ ID NO: 4 or SEQ ID NO: 6. In particular, the term "homologue" covers identity with respect to structure and/or function providing the resultant amino acid sequence has GPCR activity. With respect to sequence identity (i.e. similarity), preferably there is at least 70%, preferably at least 75%, 80%, 85%, 90%, 95% or, most preferably there is at least 95%, more preferably at least 98%, sequence identity. These terms also encompass polypeptides derived from amino acids which are allelic variations of the GPR146 GPCR nucleic acid sequence.

Where reference is made to the "receptor activity" or "biological activity" of a receptor such as GPR146 GPCR, these terms are intended to refer to the metabolic or physiological function of the GPR146 receptor, including similar activities or improved activities or these activities with decreased undesirable side effects. Also included are antigenic and immunogenic activities of the GPR146 receptor. Examples of GPCR activity, and methods of assaying and quantifying these activities, are known in the art, and are described in detail elsewhere in this document.

GPR146 polypeptides of the invention may further comprise heterologous amino acid sequences, typically at the N-terminus or C-terminus, preferably the N-terminus. Heterologous sequences may include sequences that affect intra or extracellular protein targeting (such as leader sequences). Heterologous sequences may also include sequences that increase the immunogenicity of the polypeptide of the invention and/or which facilitate identification, extraction and/or purification of the polypeptides. Another heterologous sequence that is particularly preferred is a polyamino acid sequence such as polyhistidine which is preferably N-terminal. A polyhistidine sequence of at least 10 amino acids, preferably at least 17 amino acids but fewer than 50 amino acids is especially preferred.

The GPR146 GPCR polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

GPR146 polypeptides of the invention are preferably made by recombinant means, using known techniques. However they may also be made by synthetic means using techniques well known to skilled persons such as solid phase synthesis. Polypeptides of the invention may also be produced as fusion proteins, for example to aid in extraction and purification. Examples of fusion protein partners include glutathione-S-transferase (GST), 6xHis, GAL4 (DNA-binding and/or transcriptional activation domains) and β-gatactosidase. It may also be convenient to include a proteolytic cleavage site between the fusion protein partner and the protein sequence of interest to allow removal of fusion protein sequences, such as a thrombin cleavage site. Preferably the fusion protein will not hinder the function of the protein of interest sequence.

GPR146 polypeptides of the invention may be in a substantially isolated form. This term is intended to refer to alteration by the hand of man from the natural state. If an "isolated" composition or substance occurs in nature, it has been changed or removed from its original environment, or both. For example, a polynucleotide, nucleic acid or a polypeptide naturally present in a living animal is not "isolated," but the same polynucleotide, nucleic acid or polypeptide separated from the coexisting materials of its natural state is "isolated", as the term is employed herein.

It will however be understood that the GPR146 GPCR protein may be mixed with carriers or diluents which will not interfere with the intended purpose of the protein and still be regarded as substantially isolated. A polypeptide of the invention may also be in a substantially purified form, in which case it will generally comprise the protein in a preparation in which more than 90%, for example, 95%, 98% or 99% of the protein in the preparation is a GPR146 GPCR polypeptide of the invention.

The present invention also relates to peptides comprising a portion of a GPR146 polypeptide according to the invention. Thus, fragments of GPR146 GPCR and its homologues, variants or derivatives are included. The peptides of the present invention may be between 2 and 200 amino acids, preferably between 4 and 40 amino acids in length. The peptide may be derived from a GPR146 GPCR polypeptide as disclosed here, for example by digestion with a suitable enzyme, such as trypsin. Alternatively the peptide, fragment, etc may be made by recombinant means, or synthesised synthetically.

The term "peptide" includes the various synthetic peptide variations known in the art, such as retroinverso D peptides. The peptide may be an antigenic determinant and/or a T-cell epitope. The peptide may be immunogenic *in vivo.* Preferably the peptide is capable of inducing neutralising antibodies *in vivo.*

The GPR146 polypeptides according to the invention may comprise a sequence which is conserved across species. A conserved region shows a high degree of homology between at least two species. For example, the region may show at least 60%, preferably at most 70%, preferably at least 80%, more preferably at least 90%, even more preferably at least 95% identity at the amino acid level using the tests described above. Peptides which comprise a sequence which corresponds to a conserved region may be used in therapeutic strategies as explained in further detail below. Alternatively, the GPR146 GPCR peptide may comprise a sequence which corresponds to at least part of a non-conserved region. A heterologous region shows a low degree of homology between at least two species.

### GPR146 GPCR Polynucleotides and Nucleic Acids

This invention encompasses GPR146 polynucleotides, GPR146 nucleotides and GPR146 nucleic acids, methods of production, uses of these, etc, as described in further detail elsewhere in this document.

The terms "GPR146 polynucleotide", "GPR146 nucleotide" and "GPR146 nucleic acid" may be used interchangeably, and are intended to refer to a polynucleotide/nucleic acid comprising a nucleic acid sequence as shown in SEQ ID NO: 1, SEQ ID NO:2, SEQ ID NO: 3 or SEQ ID NO: 5, or a homologue, variant or derivative thereof. Preferably, the polynucleotide/nucleic acid comprises or is a homologue, variant or derivative of the nucleic acid sequence SEQ ID NO: 1, SEQ ID NO:2 or SEQ ID NO: 3, most preferably, SEQ ID NO: 3. It should be noted that two human GPR146 cDNA sequences, identified herein as SEQ ID NO: 1 and SEQ ID NO: 2, have been identified. Both of these contain the ORF identified herein as SEQ ID NO:3. Both SEQ ID NO: 1 and SEQ ID NO: 2 are within the scope of the invention.

These terms are also intended to include a nucleic acid sequence capable of encoding a polypeptide and/or a peptide of the present invention, i.e., a GPR146 polypeptide. Thus, GPR146 GPCR polynucleotides and nucleic acids comprise a nucleotide sequence capable of encoding a polypeptide comprising the amino acid sequence shown in SEQ ID NO: 4 or SEQ ID NO: 6, or a homologue, variant or derivative thereof. Preferably, the GPR146 GPCR polynucleotides and nucleic acids comprise a nucleotide sequence capable of encoding a polypeptide comprising the amino acid sequence shown in SEQ ID NO: 4, or a homologue, variant or derivative thereof.

The term "polynucleotide" is well known in the art and to refer to any polyribonucleotide or polydeoxribonucleotide, which may be modified or unmodified RNA or DNA. "Polynucleotides" include, without limitation single- and double-stranded molecules (and molecules comprising double and single stranded regions), hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

It will be understood by the skilled person that numerous nucleotide sequences can encode the same polypeptide as a result of the degeneracy of the genetic code.

As used herein, the term "nucleotide sequence" refers to nucleotide sequences, oligonucleotide sequences, polynucleotide sequences and variants, homologues, fragments and derivatives thereof (such as portions thereof). The nucleotide sequence may be RNA or (preferably) DNA, of genomic, synthetic or recombinant origin which may be double-stranded or single-stranded whether representing the sense or antisense strand or combinations thereof.

Preferably, the resultant nucleotide sequence encodes a polypeptide having GPCR activity, preferably having at least the same activity of the GPCR shown as SEQ ID NO: 4 or SEQ ID NO: 6. Preferably, the term "homologue" refers to a polynucleotide that encodes a polypeptide which has GPCR activity. With respect to sequence identity (i.e. similarity), preferably there is at least 70%, more preferably at least 75%, more preferably at least 85%, more preferably at least 90% sequence identity. More preferably there is at least 95%, more preferably at least 98%, sequence identity. These terms also encompass allelic variations of the sequences.

### Calculation of Sequence Homology

Sequence identity with respect to any of the sequences presented here can be determined by a simple "eyeball" comparison (i.e. a strict comparison) of any one or more of the sequences with another sequence to see if that other sequence has, for example, at least 70% sequence identity to the sequence(s).

Relative sequence identity can also be determined by computer programs that can calculate % identity between two or more sequences using any suitable algorithm for determining identity, using for example default parameters. Examples of such a computer program include CLUSTAL, the GCG program package (Devereux, et al 1984 Nucleic Acids Research 12: 387), FASTA (Atschul et al 1990 J Molec Biol 403-410), and BLAST (Ausubel et al, 1999 ibid).

Preferably, the BLAST algorithm is employed, provided at http://www.ncbi.nlon,nih.gov/BLAST, with parameters set to default values (Histogram = yes; descriptions = 100; Expect - 10; Alignments = 50; matrix - BLOSUM62 (no alternative scoring matrices for BLASIN); Filter - Dust (BLASIN), SEG (other programs)). The BLAST algorithm is described in detail at http://www.ncbi.nih.gov/BLAST/blast_help.html, which is incorporated herein by reference.

### Hybridisation and Homologues

The present invention encompasses nucleotide sequences that are capable of hybridising to the sequences presented herein, or any fragment or derivative thereof, or to the complement of any of the above. Preferably, the sequence capable of hybridising is the complement of the relevant sequence.

Nucleotide sequences of the invention capable of selectively hybridising to the nucleotide sequences presented herein, or to their complement, will be generally at least 60%, preferably at least 70%, more preferably at least 75%, more preferably at least 85 or 90% and even more preferably at least 95% or 98% homologous to the corresponding nucleotide sequences presented herein over a region of at least 20, preferably at least 25 or 30, for instance at least 40, 60 or 100 or more contiguous nucleotides. Preferred nucleotide sequences of the invention will comprise regions homologous to SEQ ID NO: 1, 2, 3 or 5, preferably at least 60%, preferably at least 70%, 80% or 90% and more preferably at least 95% homologous to one of the sequences.

The term "selectively hybridizable" means that the nucleotide sequence used as a probe is used under conditions where a target nucleotide sequence of the invention is found to hybridize to the probe at a level significantly above background. Also included within the scope of the present invention are nucleotide sequences that are capable of hybridizing to the nucleotide sequences presented herein under conditions of intermediate to maximal stringency in a process familiar to those skilled in the art. Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum higher stringency hybridization can be used to identify or detect identical allows the identification of nucleotide sequences while an intermediate (or low) stringency hybridization can be used to identify or detect similar or related nucleotide sequences.with higher homologies.

### Functional Assay for GPR146 GPCR

Putative GPR146 polynucleotides may be verified by sequence analysis or functional assays. For example, the putative GPR146 GPCR or homologue may be assayed for receptor activity using methods of assaying receptor which activity are well known in the art. A preferred method is the Xenopus oocyte system using two electrode voltage clamps, as mentioned in Wagner et al, Cell Physiol Biochem. 2000; 10(1-2); 1-12. The *Xenopus* system may also be used to screen known ligands and tissue/cell extracts for activating ligands, as described in further detail below.

### Expression Assays for GPR146 GPCR

To design useful therapeutics for treating GPR146 GPCR associated diseases, it is useful to determine the expression profile of GPR146 (whether wild-type or a particular mutant). Methods known in the art may be used to determine the organs, tissues and cell types (as well as the developmental stages) in which GPR146 is expressed. For example, traditional or "electronic" (i.e. homology searching in a database) Northern blots (Sambrook, supra, ch. 7 and Ausubel, F. M. et al. supra, ch. 4 and 16.) may be conducted. Reverse-transcriptase PCR (RT-PCR) may also be employed to assay expression of the GPR146 gene or mutant. More sensitive methods for determining the expression profile of GPR146 include RNAse protection assays, as known in the art.

### Expression of GPR146 GPCR Polypeptides

Methods for producing a GPR146 GPCR polypeptide, comprise, in general, culturing a host cell comprising a nucleic acid encoding GPR146 GPCR polypeptide, or a homologue, variant, or derivative thereof, under suitable conditions (i.e., conditions in which the GPR146 GPCR polypeptide is expressed).

Methods which are well known to those skilled in the art are used to construct expression vectors containing sequences encoding GPR146 GPCR and appropriate transcriptional and translational control elements. These methods include *in vitro* recombinant DNA techniques, synthetic techniques, and *in vivo* genetic recombination. A variety of expression vector/host systems may be utilized to contain and express sequences encoding GPR146 GPCR. These will be apparent to the skilled person and include, but are not limited to, microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus (CaMV) or tobacco mosaic virus (TMV)) or with bacterial expression vectors (e.g., Ti or pBR322 plasmids); or animal cell systems in particular mammalian e.g. human cells, where a number of viral-based expression systems may be utilised. Artificial chromosomes (e.g. yeast, human) may be used to deliver large fragments of nucleic acid to a cell.

The "control elements" or "regulatory sequences" are those non-translated regions of the vector (i.e., enhancers, promoters, and 5' and 3' untranslated regions) which interact with host proteins to carry out transcription and translation. Such elements may vary in their strength and specificity. Depending on the vector system and host utilized, any number of suitable transcription and translation elements, including constitutive and inducible promoters, may be used as will be apparent to one skilled in the art.

Specific initiation signals may also be used to achieve more efficient translation of sequences encoding GPR146 GPCR. Such signals include the ATG initiation codon and adjacent sequences.. In cases where sequences encoding GPR146 GPCR and its initiation codon and upstream sequences are inserted into the appropriate expression vector, no additional transcriptional or translational control signals may be needed. However, in cases where only coding sequence, or a fragment thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers appropriate for the particular cell system used, such as those described in the literature. (Scharf, D. et al. (1994) Results Probl. Cell Differ. 20:125-162.)

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding, and/or function. Different host cells which have specific cellular machinery and characteristic mechanisms for post-translational activities (e.g., CHO, HeLa, MDCK, HEK293, and WI38), are available from the American Type Culture Collection (ATCC, Bethesda, Md.) and may be chosen to ensure the correct modification and processing of the foreign protein.

For long term, high yield production of recombinant proteins, stable expression is preferred. For example, cell lines capable of stably expressing GPR146 GPCR can be transformed using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. Resistant clones of stably transformed cells may be proliferated using tissue culture techniques appropriate to the cell type. Any number of selection systems may be used to recover transformed cell lines, as is well known in the art.

Alternatively, host cells which contain the nucleic acid sequence encoding GPR146 GPCR and express GPR146 GPCR may be identified by a variety of other procedures known to those of skill in the art. These procedures include, but are not limited to, DNA--DNA or DNA-RNA hybridizations and protein bioassay or immunoassay techniques which include membrane, solution, or chip-based technologies for the detection and/or quantification of nucleic acid or protein sequences.

Host cells transformed with nucleotide sequences encoding GPR146 GPCR may be cultured under conditions suitable for the expression and recovery of the protein from cell culture. The protein produced by a transformed cell may be located in the cell membrane, secreted or contained intracellularly depending on the sequence and/or the vector used. As will be understood by those of skill in the art, expression vectors containing polynucleotides which encode GPR146 GPCR may be designed to contain signal sequences which direct secretion of GPR146 GPCR through a prokaryotic or eukaryotic cell membrane. Other constructions may be used to join sequences encoding GPR146 GPCR to nucleotide sequences encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences, such as those specific for Factor XA or enterokinase (Invitrogen, San Diego, Calif.), between the purification domain and the GPR146 GPCR-encoding sequence may be used to facilitate purification. One such expression vector provides for expression of a fusion protein containing GPR146 GPCR and a nucleic acid encoding 6 histidine residues preceding a thioredoxin or an enterokinase cleavage site. The histidine residues facilitate purification on immobilized metal ion affinity chromatography (IMIAC; described in Porath, J. et al. (1992) Prot. Exp. Purif. 3: 263-281), while the enterokinase cleavage

### Biosensors

The GPR146 polypeptides, nucleic acids, probes, antibodies, expression vectors and ligands are useful as (and for the production of) biosensors.

According to Aizawa (1988), Anal. Chem. Symp. 17: 683, a biosensor is defined as being a unique combination of a receptor for molecular recognition, for example a selective layer with immobilized antibodies or receptors such as a GPR146 G-protein coupled receptor, and a transducer for transmitting the values measured. One group of such biosensors will detect the change which is caused in the optical properties of a surface layer due to the interaction of the receptor with the surrounding medium. Among such techniques may be mentioned especially ellipsometry and surface plasmon resonance. Biosensors incorporating GPR146 may be used to detect the presence or level of GPR146 ligands, for example, peptide hormones, nucleotides such as purines or purine analogues, or analogues of these ligands. The construction of such biosensors is well known in the art.

Thus, cell lines expressing GPR146 receptor may be used as reporter systems for detection of ligands via receptor-promoted formation of [3H]inositol phosphates or other second messengers (Watt et al., 1998, J Biol Chem May 29;273(22):14053-8). Receptor-ligand biosensors are also described in Hoffman et al., 2000, Proc Natl Acad Sci U S A Oct 10;97(21):11215-20. Optical and other biosensors comprising GPR146 may also be used to detect the level or presence of interaction with G-proteins and other proteins, as described by, for example, Figler et al, 1997, Biochemistry Dec 23;36(51):16288-99 and Sarrio et al., 2000, Mol Cell Biol.

### Screening Assays

The GPR146 polypeptide of the present invention is employed in a screening process for agents which bind the receptor and which activate (agonists) or inhibit activation of (antagonists) of GPR146. A method of identifying an agent suitable for therapy of a disorder of lipid metabolism comprises the step of determining whether a candidate agent affects the activity of GPR146. As used herein, the term "agent" refers to a moiety that could affect the activity of a receptor, as will be appreciated by one skilled in the art. Preferably, the agent is a compound. A candidate agent is therefore a substance that is tested to determine whether it (the candidate agent) affects the activity of GPR146.

The term "compound" refers to a chemical compound (naturally occurring or synthesised), such as a biological macromolecule (e.g., nucleic acid, protein, non-peptide, or organic molecule), or an extract made from biological materials such as bacteria, plants, fungi, or animal (particularly mammalian) cells or tissues, or even an inorganic element or molecule. Preferably the compound is an antibody.

Methods of determining whether a candidate agent binds to or otherwise affects the activity of a receptor (GPR146) are well known in the art. *In vitro, ex vivo* and *in silico* (i.e. the use of a docking algorithm) methods are within the scope of the invention.

Polypeptides of the invention may be used to assess the binding of agents such as small molecule substrates and ligands in, for example, cells, cell-free preparations, chemical libraries, and natural product mixtures. These substrates and ligands may be natural substrates and ligands or may be structural or functional mimetics. See Coligan et al., Currenf Protocols in Immunology 1(2):Chapter 5 (1991).

In general, GPR146 GPCR agonists and antagonists are employed for therapeutic and prophylactic purposes for disorders of lipid metabolism.

Rational design of candidate compounds likely to be able to interact with GPR146 GPCR protein may be based upon structural studies of the conformation of a polypeptide according to the invention. Preferred structural analyses include x-ray crystallography and NMR. These provide guidance as to which amino acid residues form molecular contact regions. For a detailed description of protein structural determination, see, e.g., Blundell and Johnson (1976) Protein Crystallography, Academic Press, New York.

An alternative to rational design uses a screening procedure which involves in general producing appropriate cells which express the GPR146 receptor polypeptide of the present invention on the surface thereof, and contacting the cell with a candidate agent, to determine whether the candidate agent affects the activity of the GPR146 on the surface of the cell.

Suitable cells include cells from animals, yeast, *Drosophila* or *E*. *coli.* Cells expressing the receptor (or cell membrane containing the expressed receptor) are contacted with a test compound to observe binding, stimulation or inhibition of a functional response. For example, *Xenopus* two electrodes voltage clamp system (Wagner et al, Supra) may be used. Furthermore, microphysiometric assays may be employed to assay GPR146 receptor activity. Activation of a wide variety of secondary messenger systems results in extrusion of small amounts of acid from a cell. The acid formed is largely as a result of the increased metabolic activity required to fuel the intracellular signalling process. The pH changes in the media surrounding the cell are very small but are detectable by, for example, the cytosensor microphysiometer (Molecular Devices Ltd., Menlo Park, Calif.). The cytosensor is thus capable of detecting the activation of a receptor which is coupled to an energy utilizing intracellular signaling pathway such as the G-protein coupled receptor of the present invention.

Instead of testing each candidate compound individually with the GPR146 receptor, a library or bank of candidate ligands may advantageously be produced and screened. Thus, for example; a bank of over 200 putative receptor ligands has been assembled for screening. The bank comprises: transmitters, hormones and chemokines known to act via a human 7TM receptor; naturally occurring compounds which may be putative agonists for a human 7TM receptor; non-mammalian, biologically active peptides for which a mammalian counterpart has not yet been identified; and compounds not found in nature, but which activate 7TM receptors with unknown natural ligands. This bank is used to screen the receptor for known ligands, using both functional (i.e. calcium, cAMP, microphysiometer, oocyte electrophysiology, etc, see elsewhere) as well as binding assays as described in further detail elsewhere. However, a large number of mammalian receptors exist for which there remains, as yet, no agonist or antagonist. Thus, active ligands for these receptors may not be included within the ligands banks as identified to date. Accordingly, theGPR146 receptor of the invention is also functionally screened (using calcium, cAMP, microphysiometer, oocyte electrophysiology, etc., functional screens) against tissue extracts to identify natural ligands. Extracts that produce positive functional responses can be sequentially subfractionated, with the fractions being assayed as described here, until an activating ligand is isolated and identified.

7TM receptors which are expressed in HEK 293 cells have been shown to be coupled functionally to activation of PLC and calcium mobilization and/or cAMP stimuation or inhibition. One screening technique therefore includes the use of cells which express the GPR146 GPCR of this invention (for example, transfected *Xenopus* oocytes, CHO or HEK293 cells) in a system which measures extracellular pH or intracellular calcium changes caused by receptor activation. In this technique, compounds may be contacted with cells expressing the receptor polypeptide of the present invention. A second messenger response, e.g., signal transduction, pH changes, or changes in calcium level, is then measured to determine whether the potential compound activates or inhibits the receptor.

Another method involves screening for receptor inhibitors by determining inhibition or stimulation of GPR146 receptor-mediated cAMP and/or adenylate cyclase accumulation. Such a method involves transfecting a eukaryotic cell with the receptor of this invention to express the receptor on the cell surface. The cell is then exposed to potential antagonists in the presence of the receptor of this invention. The amount of cAMP accumulation is then measured. If the potential antagonist binds the receptor, and thus inhibits receptor binding, the levels of receptor-mediated cAMP, or adenylate cyclase, activity will be reduced or increased.

Another method for detecting agonists or antagonists for the receptor of the present invention is the yeast based technology as described in U.S. Pat. No. 5,482,835.

Where the candidate compounds are proteins, in particular antibodies or peptides, libraries of candidate compounds may be screened using phage display techniques. Phage display is a protocol of molecular screening which utilises recombinant bacteriophage. The technology involves transforming bacteriophage with a gene that encodes one compound from the library of candidate compounds, such that each phage or phagemid expresses a particular candidate compound. The transformed bacteriophage (which preferably is tethered to a solid support) expresses the appropriate candidate compound and displays it on their phage coat. Specific candidate compounds which are capable of binding to a polypeptide or peptide of the invention are enriched by selection strategies based on affinity interaction. The successful candidate agents are then characterised. Phage display has advantages over standard affinity ligand screening technologies. The phage surface displays the candidate agent in a three-dimensional configuration, more closely resembling its naturally occurring conformation. This allows for more specific and higher affinity binding for screening purposes.

Another method of screening a library of compounds utilises eukaryotic or prokaryotic host cells which are stably transformed with recombinant DNA molecules expressing a library of compounds. Such cells, either in viable or fixed form, can be used for standard binding-partner assays. See also Parce et al. (1989) Science 246:243-247; and Owicki et al. (1990) Proc. Nat'I Acad. Sci. USA 87;4007-4011, which describe sensitive methods to detect cellular responses. Competitive assays are particularly useful, where the cells expressing the library of compounds are contacted or incubated with a labelled antibody known to bind to a GPR146 polypeptide of the present invention, such as ¹²⁵I-antibody, and a test sample such as a candidate compound whose binding affinity to the binding composition is being measured. The bound and free labelled binding partners for the polypeptide are then separated to assess the degree of binding. The amount of test sample bound is inversely proportional to the amount of labelled antibody binding to the polypeptide.

Any one of numerous techniques can be used to separate bound from free binding partners to assess the degree of binding. This separation step could typically involve a procedure such as adhesion to filters followed by washing, adhesion to plastic following by washing, or centrifugation of the cell membranes.

Still another approach is to use solubilized, unpurified or solubilized purified polypeptide or peptides, for example extracted from transformed eukaryotic or prokaryotic host cells. This allows for a "molecular" binding assay with the advantages of increased specificity, the ability to automate, and high drug test throughput. In a preferred embodiment, the GPR146 polypeptide is contacted with a candidate agent to determine whether the candidate agent affects the activity of the GPR146.

Another technique for candidate compound screening involves an approach which provides high throughput screening for new compounds having suitable binding affinity, e.g., to a polypeptide of the invention, and is described in detail in WO84/03564. First, large numbers of different small peptide test compounds are synthesized on a solid substrate, e.g., plastic pins or some other appropriate surface; see Fodor *et al.* (1991). Then all the pins are reacted with solubilized polypeptide of the invention and washed. The next step involves detecting bound polypeptide. Compounds which interact specifically with the polypeptide will thus be identified.

Ligand-binding assays provide a direct method for ascertaining receptor pharmacology and are adaptable to a high throughput format. The purified ligand for a receptor may be radiolabeled to high specific activity (50-2000 Ci/mmol) for binding studies. A determination is then made that the process of radiolabeling does not diminish the activity of the ligand towards its receptor. Assay conditions for buffers, ions, pH and other modulators such as nucleotides are optimized to establish a workable signal to noise ratio for both membrane and whole cell receptor sources. For these assays, specific receptor biding is defined as total associated radioactivity minus the radioactivity measured in the presence of an excess of unlabeled competing ligand. Where possible, more than one competing ligand is used to define residual nonspecific binding.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed.

Further, the assays may simply comprise the steps of mixing a candidate compound with a solution containing a GPR146 GPCR polypeptide to form a mixture, measuring GPR146 GPCR activity in the mixture, and comparing the GPR146 GPCR activity of the mixture to a standard.

The GPR146 GPCR cDNA, protein and antibodies to the protein may also be used to configure assays for detecting the effect of added compounds on the production of GPR146 GPCR mRNA and protein in cells. For example, an ELISA may be constructed for measuring secreted or cell associated levels of GPR146 GPCR protein using monoclonal and polyclonal antibodies by standard methods known in the art, and this can be used to discover agents which may inhibit or enhance the production of GPR146 GPCR (also called antagonist or agonist, respectively) from suitably manipulated cells or tissues. Standard methods for conducting screening assays are well understood in the art.

Examples of potential GPR146 GPCR antagonists include antibodies or, in some cases, nucleotides and their analogues, including purines and purine analogues, oligonucleotides or proteins which are closely related to the ligand of the GPR146 GPCR, e.g., a fragment of the ligand, or small molecules which bind to the receptor but do not elicit a response, so that the activity of the receptor is prevented.

### Transgenic Animals

The present invention further encompasses transgenic animals capable of expressing natural or recombinant GPR146 GPCR, or a homologue, variant or derivative, at elevated or reduced levels compared to the normal expression level. Included are transgenic animals (GPR146 knockouts) which do not express a functional GPR146 receptor as a result of one or more loss of function mutations, including a deletion (of both alleles i.e. -/-), of the GPR146 gene. These animals are said to have a functionally-disrupted endogenous GPR146 gene. A non-human animal that does not express any functional (GPR146) polypeptide is often referred to as having a "null" mutation.

The transgenic animal of the invention is a non-human mammal, such as a pig, a sheep or a rodent. Most preferably the transgenic animal is a mouse or a rat. Such transgenic animals may be used in screening procedures to identify agonists and/or antagonists of GPR146 GPCR, as well as to test for their efficacy as treatments for diseases *in vivo.*

Whether a candidate agent affects the activity of GPR146 can be determined using a non-human animal, as discussed in more detail below. It should be noted that the animal used in such an assay can be wild type or transgenic, as will be appreciated by the skilled person.

For example, transgenic animals that have been engineered to be deficient in the production of GPR146 GPCR may be used in assays to identify agonists and/or antagonists of GPR146 GPCR. One assay is designed to evaluate a potential drug (a candidate ligand or compound) to determine if it produces a physiological response in the absence of GPR146 GPCR receptors. This may be accomplished by administering the drug to a transgenic animal as discussed above, and then assaying the animal for a particular response, which will be apparent to the skilled person. If the drug affects the activity of GPR146, a change in lipid metabolism could be expected. Tissues derived from the GPR146 knockout animals may be used in receptor binding assays to determine whether the potential drug (a candidate agent, ligand or compound) binds to the GPR146 receptor. Such assays can be conducted by obtaining a first receptor preparation from the transgenic animal engineered to be deficient in GPR146 receptor production and a second receptor preparation from a source known to bind any identified GPR146 ligands or compounds. In general, the first and second receptor preparations will be similar in all respects except for the source from which they are obtained. For example, if brain tissue from a transgenic animal (such as described above and below) is used in an assay, comparable brain tissue from a normal (wild type) animal is used as the source of the second receptor preparation. Each of the receptor preparations is incubated with a ligand known to bind to GPR146 receptors, both alone and in the presence of the candidate ligand or compound. Preferably, the candidate ligand or compound will be examined at several different concentrations.

The extent to which binding by the known ligand is displaced by the test compound is determined for both the first and second receptor preparations. Tissues derived from transgenic animals may be used in assays directly or the tissues may be processed to isolate membranes or membrane proteins, which are themselves used in the assays. A preferred transgenic animal is the mouse. The ligand may be labeled using any means compatible with binding assays. This would include, without limitation, radioactive, enzymatic, fluorescent or chemiluminescent labeling (as well as other labelling techniques as described in further detail above).

Furthermore, agents which affect the activity of GPR146 may be identified by administering candidate compounds to wild type animals expressing functional GPR146, and identifying animals which exhibit any of the phenotypic characteristics associated with altered lipid metabolism.

Methods for generating a non-human transgenic animal are well known in the art and any suitable method may be used. In an exemplary embodiment, the transgenic non-human animals of the invention are produced by introducing transgenes into the germline of the non-human animal. One or several copies of the construct may be incorporated into the genome. Embryonal target cells at various developmental stages can be used to introduce transgenes. Different methods are used depending on the stage of development of the embryonal target cell. Introduction of the transgene into the embryo can be accomplished by any means known in the art such as, for example, microinjection, electroporation, or lipofection. Following introduction of the transgene construct into the fertilized egg, the egg may be incubated in vitro for varying amounts of time, or reimplanted into the surrogate host, or both. *In vitro* incubation to maturity is within the scope of this invention. One common method in to incubate the embryos in vitro for about 1-7 days, depending on the species, and then reimplant them into the surrogate host.

For the purposes of this invention a zygote is essentially the formation of a diploid cell which is capable of developing into a complete organism. Generally, the zygote will be comprised of an egg containing a nucleus formed, either naturally or artificially, by the fusion of two haploid nuclei from a gamete or gametes.

The number of copies of the transgene constructs which are added to the zygote is dependent upon the total amount of exogenous genetic material added and will be the amount which enables the genetic transformation to occur. Theoretically only one copy is required; however, generally, numerous copies are utilized, for example, 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences.

Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Reimplantation is accomplished using standard methods. Usually, the surrogate host is anesthetized, and the embryos are inserted into the oviduct. The number of embryos implanted into a particular host will vary by species, but will usually be comparable to the number of off spring the species naturally produces.

Transgenic offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Progeny of the transgenic animals may be obtained by mating the transgenic animal with a suitable partner, or by in vitro fertilization of eggs and/or sperm obtained from the transgenic animal. Where mating with a partner is to be performed, the partner may or may not be transgenic and/or a knockout; where it is transgenic, it may contain the same or a different transgene, or both. Alternatively, the partner may be a parental line. Where in vitro fertilization is used, the fertilized embryo may be implanted into a surrogate host or incubated in vitro, or both. Using either method, the progeny may be evaluated for the presence of the transgene using methods described above, or other appropriate methods.

The transgenic animals produced in accordance with the present invention will include exogenous genetic material. As set out above, the exogenous genetic material will, in certain embodiments, be a DNA sequence which results in the production of a GPR146 GPCR receptor. Further, in such embodiments the sequence will be attached to a transcriptional control element, e.g., a promoter, which preferably allows the expression of the transgene product in a specific type of cell.

An alternative target cell for transgene introduction is the embryonal stem cell (ES). ES cells are obtained from pre-implantation embryos cultured in vitro and fused with embryos (Evans et al. (1981) Nature 292:154-156; Bradley et al. (1984) Nature 309:255-258; Gossler et al. (1986) PNAS 83: 9065-9069; and Robertson et al. (1986) Nature 322:445-448). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, R. (1988) Science 240:1468-1474.

We also provide for non-human transgenic animals, where the transgenic animal is characterized by having functionally-disrupted endogenous GPR146 gene, preferably as described above, as models for GPR146 receptor function. Alterations to the gene include deletions or other loss of function mutations, introduction of an exogenous gene having a nucleotide sequence with targeted or random mutations, introduction of an exogenous gene from another species, or a combination thereof. The transgenic animals may be either homozygous or heterozygous for the alteration, preferably heterozygous. The animals and cells derived therefrom are useful for screening biologically active agents that affect the activity of GPR146. The screening methods are of particular use for determining the specificity and action of potential therapies for disorders of lipid metabolism.

The present invention also provides for a GPR146 GPCR-deficient transgenic non-human animal (a "GPR146 GPCR knock-out"). Such an animal is one which expresses lowered or no GPR146 GPCR activity, preferably as a result of an endogenous GPR146 GPCR genomic sequence being disrupted or deleted. Preferably, such an animal expresses no GPCR activity. More preferably, the animal expresses no activity of the GPR146 GPCR shown as SEQ ID NO: 4 or SEQ ID NO: 6. GPR146 GPCR knock-outs may be generated by various means known in the art, as described in further detail below.

A GPR146 GPCR deficient transgenic animal may be generated as described in Examples 1 and 2.

### Antibodies

For the purposes of this invention, the term "antibody", unless specified to the contrary, includes but is not limited to, polyclonal, monoclonal, chimeric, single chain, Fab fragments and fragments produced by a Fab expression library. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. The antibodies and fragments thereof may be humanised antibodies, for example as described in EP-A-0239400. Furthermore, antibodies with fully human variable regions (or their fragments), for example, as described in US Patents Nos. 5,545,807 and 6,075,181 may also be used. Neutralizing antibodies, i.e., those which inhibit biological activity of the substance amino acid sequences, are especially preferred for diagnostics and therapeutics.

Antibodies may be produced by standard techniques, such as by immunisation or by using a phage display library.

A polypeptide or peptide of the present invention may be used to develop an antibody by known techniques. Such an antibody may be capable of binding specifically to the GPR146 GPCR protein or homologue, fragment, etc.

If polyclonal antibodies are desired, a selected mammal (e.g., mouse, rabbit, goat, horse, etc.) may be immunised with an immunogenic composition comprising a polypeptide or peptide of the present invention. Depending on the host species, various adjuvants, including Freunds Adjuvants and Aluminium Hydroxid, may be used to increase immunological response. BCG (*Bacilli Calmette-Guerin*) and *Corynebacterium parvum* are potentially useful human adjuvants which may be employed if purified the substance amino acid sequence is administered to immunologically compromised individuals for the purpose of stimulating systemic defence.

Monoclonal antibodies directed against epitopes obtainable from a polypeptide or peptide of the present invention can also be readily produced by one skilled in the art. The general methodology for making monoclonal antibodies by hybridomas is well known.

In addition, techniques developed for the production of "chimeric antibodies", the splicing of mouse antibody genes to human antibody genes to obtain a molecule with appropriate antigen specificity and biological activity can be used (Morrison et al (1984) Proc Natl Acad Sci 81:6851-6855; Neuberger et al (1984) Nature 312:604-608; Takeda et al (1985) Nature 314:452-454). Alternatively, techniques described for the production of single chain antibodies (US Patent No. 4,946,779) can be adapted to produce the substance specific single chain antibodies.

Antibodies, both monoclonal and polyclonal, which are directed against epitopes obtainable from a polypeptide or peptide of the present invention are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the substance and/or agent against which protection is desired. Techniques for raising anti-idiotype antibodies are known in the art. These anti-idiotype antibodies may also be useful in therapy.

The above-described antibodies may be employed to isolate or to identify clones expressing the polypeptide or to purify the polypeptides by affinity chromatography.

Antibodies against GPR146 GPCR polypeptides may also be employed to treat dyslipidaemias.

### Diagnostic Assays

GPR146 is implicated in disorders of lipid metabolism. Therefore, detecting the presence and/or activity of GPR146 in an individual, or preferably in a sample isolated from an individual, allows a diagnosis to be made on the presence of or susceptibility to a disorder of lipid metabolism, as will be apparent to one skilled in the art.

This invention also relates to the use of GPR146 GPCR polynucleotides and polypeptides (as well as homologues, variants and derivatives thereof) for use in diagnosis as diagnostic reagents or in genetic analysis. Nucleic acids complementary to or capable of hybridising to GPR146 GPCR nucleic acids (including homologues, variants and derivatives), as well as antibodies against GPR146 polypeptides are also useful in such assays.

The diagnosis is performed *in vitro,* i.e. it is not performed on or in the body of a person or animal that is being diagnosed.

A method of determining the presence of, or susceptibility to, a disorder of lipid metabolism in an individual (i.e. a patient) comprises the steps of (i) determining the expression level of GPR146 gene in a sample isolated from the individual, and (ii) determining, compared to a control, whether the individual from which the sample was isolated has or is susceptible to a disorder of lipid metabolism.

Detection of a mutated form of the GPR146 GPCR gene associated with a dysfunction will provide a diagnostic tool that can add to or define a diagnosis of a disease or susceptibility to a disease which results from under-expression, over-expression or altered expression of GPR146 GPCR. Individuals carrying mutations in the GPR146 GPCR gene (including control sequences) may be detected at the DNA level by a variety of techniques that are known in the art. Preferably, the region of interest (comprising GPR146 GPCR) is amplified using PCR, prior to mutation analysis by direct sequencing or other commonly used methods.

For example, DNA may be isolated from a patient and the DNA polymorphism pattern of GPR146 determined. The identified pattern is compared to controls of patients known to be suffering from a disease associated with over-, under- or abnormal expression of GPR146. Patients expressing a genetic polymorphism pattern associated with GPR146 associated disease may then be identified. Genetic analysis of the GPR146 GPCR gene may be conducted by any technique known in the art. For example, individuals may be screened by determining DNA sequence of a GPR146 allele, by RFLP (Restriction Fragment Length Polymorphism) or SSCP (Single Strand Conformation Polymorphism) analysis. Patients may be identified as having a genetic predisposition for a disease associated with the over-, under-, or abnormal expression of GPR146 by detecting the presence of a DNA polymorphism in the gene sequence for GPR146 or any sequence controlling its expression.

Patients so identified can then be treated to prevent the occurrence of disorders of lipid metabolism or more aggressively in the early stages of the disorder of lipid metabolism to prevent the further occurrence or development of the disease.

The diagnostic assays offer a process for diagnosing or determining a susceptibility to disorders of lipid metabolism through detection of mutation in the GPR146 gene by the methods described.

The presence of GPR146 polypeptides and nucleic acids may be detected in a sample. Thus, infections and diseases as listed above can be diagnosed by methods comprising determining from a sample derived from a subject an abnormally decreased or increased level of the GPR146 polypeptide or GPR146 mRNA. The sample may comprise a cell or tissue sample from an organism suffering or suspected to be suffering from a disease associated with increased, reduced or otherwise abnormal GPR146 expression, including spatial or temporal changes in level or pattern of expression. The level or pattern of expression of GPR146 in an organism suffering from or suspected to be suffering from such a disease may be usefully compared with the level or pattern of expression in a normal organism as a means of diagnosis of disease.

In general therefore, the invention includes a method of detecting the presence of a nucleic acid comprising a GPR146 GPCR nucleic acid in a sample, by contacting the sample with at least one nucleic acid probe which is specific for said nucleic acid and monitoring said sample for the presence of the nucleic acid. For example, the nucleic acid probe may specifically bind to the GPR146 nucleic acid, or a portion of it, and binding between the two detected; the presence of the complex itself may also be detected. Furthermore, the invention encompasses a method of detecting the presence of a GPR146 polypeptide by contacting a cell sample with an antibody capable of binding the polypeptide and monitoring said sample for the presence of the polypeptide. This may conveniently be achieved by monitoring the presence of a complex formed between the antibody and the polypeptide, or monitoring the binding between the polypeptide and the antibody. Methods of detecting binding between two entities are known in the art, and include FRET (fluorescence resonance energy transfer), surface plasmon resonance, etc.

Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as a GPR146 GPCR, in a sample derived from a host are well-known to those of skill in the art. Such assay methods include radioimmunoassays, competitive-binding assays, Western Blot analysis and ELISA assays.

### Prophylactic and Therapeutic Methods

This invention provides methods of treating an abnormal conditions related to both an excess of and insufficient amounts of GPR146 GPCR activity.

An agent that affects the activity of a GPR146 polypeptide can be used to manufacture a medicament for the prevention or treatment of a disorder of lipid metabolism.

If the activity of GPR146 is in excess, several approaches are available. One approach comprises administering to a subject an inhibitor compound (antagonist) as hereinabove described along with a pharmaceutically acceptable carrier in an amount effective to inhibit activation by blocking binding of ligands to the GPR146 GPCR, or by inhibiting a second signal, and thereby alleviating the abnormal condition.

In another approach, soluble forms of GPR146 polypeptides still capable of binding the ligand in competition with endogenous GPR146 may be administered. Typical embodiments of such competitors comprise fragments of the GPR146 polypeptide.

In still another approach, expression of the gene encoding endogenous GPR146 can be inhibited using expression blocking techniques. Known such techniques involve the use of exogenous polynucleotides such as antisense sequences, either internally generated or separately administered. See, for example, O'Connor, J Neurochem (1991) 56:560 in Oligodeoxvnucleotides as Antisense Inhibitors of Gene Expression, CRC Press, Boca Raton, Fla. (1988). Alternatively, oligonucleotides which form triple helices with the gene can be supplied. See, for example, Lee et al., Nucleic Acids Res (1979) 6:3073; Cooney et al., Science (1988) 241:456; Dervan et al., Science (1991) 251:1360. These oligomers can be administered per se or the relevant oligomers can be expressed in vivo.

For treating abnormal conditions related to an under-expression of GPR146 and its activity, several approaches are also available. One approach comprises administering to a subject a therapeutically effective amount of a compound which activates GPR146, i.e. an agonist as described above, in combination with a pharmaceutically acceptable carrier, to thereby alleviate the abnormal condition. Alternatively, gene therapy may be employed to effect (preferably increase) the endogenous production of GPR146 by the relevant cells in the subject. In a preferred embodiment, an exogenous (originating outside of the host, preferably originating from a different species) GPR 146 polynucleotide is used in the manufacture of a medicament for the treatment or prevention of a disorder of lipid metabolism. For example, an exogenous GPR146 polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells in vivo and expression of the polypeptide in vivo. For overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996).

### Formulation and Administration

Peptides, such as the soluble form of GPR146 GPCR polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

### EXAMPLES

### Example 1. Transgenic GPR146 knockout mouse: Construction of GPR146 Gene Targeting Vector

The GPR146 gene was identified bio-informatically using homology searches of genome databases. A ∼300kb murine genomic contig was assembled from various databases. This contig provided sufficient flanking sequence information to enable the design of homologous arms to clone into the targeting vector.

The murine GPR146 gene has 1 coding exon. The targeting strategy is designed to remove the majority of the coding sequence, including all of the 7tm region. A 4.0kb 5' homologous arm and a 1.8kb 3' homologous arm flanking the region to be deleted are amplified by PCR and the fragments are cloned into the targeting vector. The 5' end of each oligonucleotide primer used to amplify the arms is synthesised to contain a different recognition site for a rare-cutting restriction enzyme, compatible with the cloning sites of the vector polylinkers situated at either end of the reporter/selection cassette, and absent from the arms themselves. In the case of GPR146, the primers are designed as listed in the primer table below, with 5' arm cloning sites of NotI/SpeI and 3'arm cloning sites of Ascl/Fsel.

In addition to the arm primer pairs (5'armF/5'armR) and (3'armF/3'armR), further primers specific to the GPR146 locus are designed for the following purposes: 5' and 3' probe primer pairs (5'prF/5'prR and 3'prF/3'prR) to amplify two short 150-300bp fragments of non-repetitive genomic DNA external to and extending beyond each arm, to allow Southern analysis of the targeted locus, in isolated putative targeted clones; a mouse genotyping primer pair (hetF and hetR) which allows differentiation between wild-type, heterozygote and homozygous mice, when used in a multiplex PCR with a vector specific primer, in this case, Asc403; and lastly, a target screening primer (3'scr) which anneals downstream of the end of the 3' arm region, and which produces a target event specific 1.9kb amplimer when paired with a primer specific to the 3' end of the vector, in this case Asc79. This amplimer can only be derived from template DNA from cells where the desired genomic alteration has occurred and allows the identification of correctly targeted cells from the background of clones containing randomly integrated copies of the vector. The location of these primers and the genomic structure of the regions of the GPR146 locus used in the targeting strategy is shown in SEQ ID NO:7 (and Figure 3).

**Table 1. GPR146 Primer Sequences**

| | |
|---|---|
| musGPR146 5'prF SEQ ID NO:8 | CCCCGTCACTGGACAAACTCAGGCATC |
| musGPR146 5'prR SEQ ID NO:9 | CTGTTCCCCTTGCGGCTGGAGAAGATG |
| musGPR146 5'armF Not SEQ ID NO:10 | tttgcggccgCCTTCTGTGGGTAGGGAGAACAGTTAG |
| musGPR146 5'armR Spe SEQ ID NO:11 | tttactagtCTGTTGAGTGGGCCACAGCTCCACATG |
| musGPR146 3'armF Asc SEQ ID NO:12 | aaaggcgcgcCTCTACCGTTACATCAACAAAGCCTTC |
| musGPR146 3'armR Fse SEQ ID NO:13 | aaaggccggccATAAAACGGTCCCCAGGGCAAAGACTG |
| musGPR146 3'scr a79 SEQ ID NO:14 | TCTCTGGACCTCTGCCTGGAGTAAGTG |
| musGPR146 3'prF SEQ ID NO:15 | TCCAAGTGGGGTTGTACGCCCTAGAAC |
| musGPR146 3'prR SEQ ID NO:16 | GGGTCTCTACAGCAAAGGGGTCAGCAC |
| musGPR146 heft SEQ ID NO:17 | CTCATCTCGAGAATCGGGAAGGAAGAC |
| musGPR146 hetR a403 SEQ ID NO:18 | GAAGGCTTTGTTGATGTAACGGTAGAG |
| Asc403 SEQ ID NO:19 | CAGCCGAACTGTTCGCCAGGCTCAAGG |
| Asc79 SEQ ID NO:20 | AACGCACGGGTGTTGGGTCGTTTGTTC |

The position of the homology arms is chosen to functionally disrupt the GPR146 gene. A targeting vector is prepared where the GPR146 region to be deleted is replaced with non-homologous sequences composed of an endogenous gene expression reporter (a frame independent lacZ gene) upstream of a selection cassette composed of a promoted neomycin phosphotransferase (neo) gene arranged in the same orientation as the GPR146 gene.

Once the 5' and 3' homology arms have been cloned into the targeting vector, a large highly pure DNA preparation is made using standard molecular biology techniques. 20 µg of the freshly prepared endotoxin-free DNA is restricted with another rare-cutting restriction enzyme Pmel, present at a unique site in the vector backbone between the ampicillin resistance gene and the bacterial origin of replication. The linearized DNA is then precipitated and resuspended in 100 µl of Phosphate Buffered Saline, ready for electroporation.

24 hours following electroporation the transfected cells are cultured for 9 days in medium containing 200µg/ml neomycin. Clones are picked into 96 well plates, replicated and expanded before being screened by PCR (using primers 3'scr and Asc79, as described above) to identify clones in which homologous recombination has occurred between the endogenous GPR146 gene and the targeting construct. Positive clones can be identified at a rate of 1 to 5%. These clones are expanded to allow replicas to be frozen and sufficient high quality DNA to be prepared for Southern blot confirmation of the targeting event using the external 5' and 3' probes prepared as described above, all using standard procedures (Russ et al, Nature 2000 Mar 2;404(6773):95-99). When Southern blots of DNA digested with diagnostic restriction enzymes are hybridized with an external probe, homologously targeted ES cell clones are verified by the presence of a mutant band as well an unaltered wild-type band. For instance, using the 5' probe, AvrII digested genomic DNA will give a 13.9kb wild-type band and a 7.8kb targeted band; and with the 3' probe, AvrII cut DNA will give a 13.9kb wild-type band and a 10kb targeted band.

### Example 2. Transgenic GPR146 knockout mouse: Generation of GPR146 Deficient Mice

C57BU6 female and male mice are mated and blastocysts are isolated at 3.5 days of gestation. 10-12 cells from a chosen clone are injected per blastocyst and 7-8 blastocysts are implanted in the uterus of a pseudopregnant F1 female. A litter of chimeric pups are born containing several high level (up to 100%) agouti males (the agouti coat colour indicates the contribution of cells descended from the targeted clone). These male chimeras are mated with female MF1 and 129 mice, and germline transmission is determined by the agouti coat colour and by PCR genotyping respectively.

PCR Genotyping is carried out on lysed tail clips, using the primers hetF and hetR with a third, vector specific primer (Asc403). This multiplex PCR allows amplification from the wild-type locus (if present) from primers hetF and hetR giving a 282bp band. The site for hetF is deleted in the knockout mice, so this amplification will fail from a targeted allele. However, the Asc403 primer will amplify a 431 bp band from the targeted locus, in combination with the hetR primer which anneals to a region just inside the 3' arm. Therefore, this multiplex PCR reveals the genotype of the litters as follows: wild-type samples exhibit a single 282 bp band; heterozygous DNA samples yield two bands at 282 bp and 431bp; and the homozygous samples will show only the target specific 431 bp band.

### Example 3. Serum Cholesterol Measurements

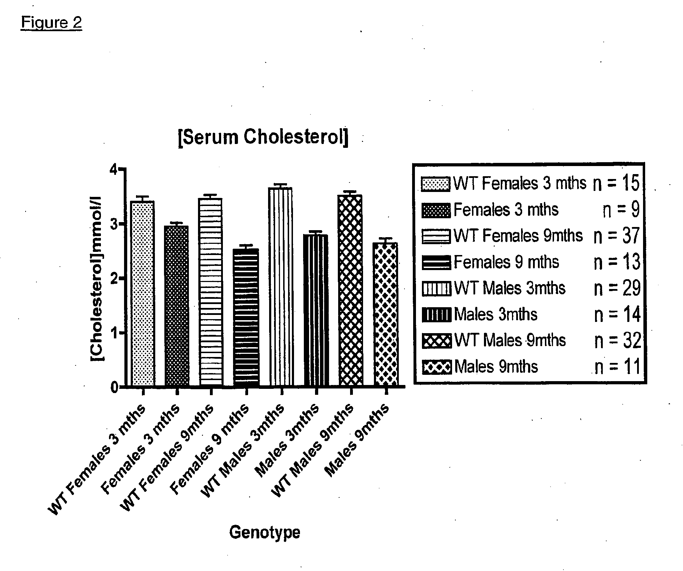

The serum cholesterol level of Wild Type and GPR146 GPCR knockout mice of different ages and sexes was assayed and the results are shown in Figure 2. The reduction in serum cholesterol levels in the GPR146 GPCR knockout mice is significant (Student's T test p<0.05) for both male and female mice.

To assay the serum cholesterol level, blood was sampled from the tail vein after prewarming the animals. Heparinized blood was spun in a table top centrifuge at 10000 rpm for 10 min. Plasma was stored frozen until measurement.

| | |
|---|---|
| Serum Asssay Method : | Enzyme assay - Cholesterol esterase |
| Reagent supplier | Dade-Behring |
| Analyser : | Dade-Behring Dimension RXL |
| Sample volume : | 3µl |
| Assay range : | 1.3 - 15.5 mmol/L |

The serum cholesterol assays were performed according Rautela, GS & Liedtke, RJ. Automated enzymic measurement of total cholesterol in serum. Clin Chem 1978; 24, which is an automated version of to Stadtman TC, Methods In Enzymology Vol III 1957; 392-394, 678-681.

Table 2 shows the principle of the assay method, using a Du Pont Automated Clinical Analyser (aca). Cholesterol esters are hydrolyzed by cholesterol esterase to produce fatty acids and free cholesterol. The latter, along with the pre-existing free cholesterol, is oxidized in a reaction catalyzed by cholesterol oxidase. The oxidation products are cholest-4-en-3-one and hydrogen peroxide. The hydrogen peroxide is reduced to water with concomitant oxidation of N,N-diethylaniline hydrochloride and 4-aminoantipyrine, which then react to form a quinoneimine dye (chromophore), Amax = 553nm, a reaction catalyzed by horseradish peroxidase. The absorbance due to the chromophore measured at 540minus the absorbance at 600 nm is directly proportional to the concentration of cholesterol. The wavelength combination of 553 minus 600 nm is not available for use in the aca, but an alternative combination that is available, 540 minus 600 nm, provides adequate (about93% as much) sensitivity. With the aca, 261.5 s area available for completion of the above reaction-that is the interval between starting the reaction and the final absorbance measurement.

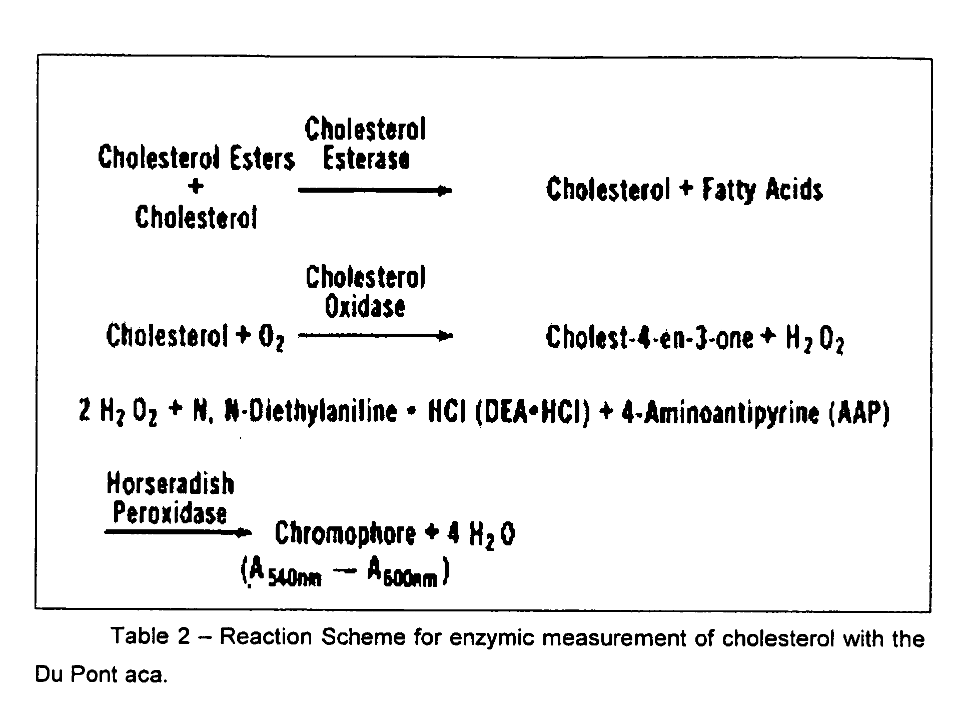

Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. An *in vitro* or *ex vivo* method of identifying an agent suitable for therapy of a disorder of lipid metabolism selected from: atheromatous disease, atherosclerosis, coronary heart disease, gall bladder disease, impaired glucose tolerance, metabolic syndrome X, restenosis, septic shock, stroke, angina pectoris, transient ischemic attacks (TIAs) or peripheral artery disease (PAD), comprising the step of determining whether a candidate agent affects the serum cholesterol level controlling activity of GPR146, wherein GPR146 comprises or encodes a polypeptide identified herein as SEQ ID No. 4 or SEQ ID No. 6, or a sequence with at least 70% sequence identity thereto.

2. A method according to claim 1, wherein the agent is an antagonist that inhibits the serum cholesterol level controlling activity of GPR146.

3. A method according to claim 1, wherein the agent is an agonist that enhances the serum cholesterol level controlling activity of GPR146.

4. A method according to any preceding claim, wherein the GPR146 consists of or encodes a polypeptide identified herein as SEQ ID No 4, SEQ ID No 6, or a sequence with at least 70% identity thereto.

5. A method according to any preceding claim, wherein the GPR146 polypeptide is contacted with the candidate agent to determine whether the candidate affects the serum cholesterol level controlling activity of the GPR146.

6. A method according to any preceding claim, wherein the candidate agent is contacted with a cell expressing a GPR146 polypeptide.

7. A method according to any preceding claim, wherein the step of determining whether the agent affects the activity of GP146 is carried out on a sample obtainable from a non-human animal expressing the GPR146 polypeptide having been administered with the candidate agent, and determining from the sample whether the animal exhibits altered lipid metabolism.

8. A method according to claim 7, wherein the non-human animal expresses a functional GPR146 polypeptide.

9. A method according to claim 7 or claim 8, wherein the non-human animal is wild-type.

10. A method according to any of claims 7 to 9, wherein the non-human animal is a rodent.

11. A method according to any preceding claim, wherein the disorder is atherosclerosis.

12. A non-human transgenic animal having a functionally-disrupted endogenous GPR146 gene, wherein the GPR146 gene is as defined in claim 1.

13. A non-human transgenic animal according to claim 12, wherein the animal has a null mutation.

14. A non-human transgenic animal according to claim 12 or claim 13, wherein the animal is -/- for the GPR146 gene.

15. A method of determining the presence of, or susceptibility to, a disorder defined in claim 1 in an individual, comprising the steps of (i) determining the expression level of GPR146 gene in a sample isolated from the patient, and (ii) determining, compared to a control, whether the individual from which the sample was isolated has or is susceptible to a disorder defined in claim 1, wherein the GPR146 gene is as defined in claim 1.

16. Use of an exogenous GPR146 polynucleotide as defined in claim 1 in the manufacture of a medicament for the treatment or prevention of a disorder defined in claim 1.

## Patentansprüche

1. Eine *in vitro* oder *ex vivo* Methode zur Identifizierung eines Wirkstoffs, der sich zur Behandlung einer Störung des Lipidmetabolismus bei folgenden eignet, nämlich atheromatöse Erkrankung, Atherosklerose, koronare Herzerkrankung, Gallenblasenerkrankung, gestörte Glukosetoleranz, metabolisches Syndrom X, Restenose, septischer Schock, Schlaganfall, Angina pectoris, transiente ischämische Attacke (TIA) oder periphere Arterienerkrankung (PAD), und den Schritt der Bestimmung umfasst, ob ein Kandidatenwirkstoff die regulierende Wirkung des GPR146 auf den Serumcholesterinspiegel beeinträchtigt, wobei GPR146 aus einem Polypeptid besteht oder dieses kodiert, das hierin als SEQ ID-Nr. 4 oder SEQ ID-Nr. 6 oder als eine Sequenz mit mindestens 70% Seqzuenzidentität zu diesem identifiziert wird.

2. Eine Methode entsprechend Anspruch 1, wobei es sich beim Wirkstoff um einen Antagonisten handelt, der die regulierende Wirkung von GPR146 auf den Serumcholesterinspiegel inhibiert.

3. Eine Methode entsprechend Anspruch 1, wobei es sich beim Wirkstoff um einen Agonisten handelt, der die regulierende Wirkung von GPR146 auf den Serumcholesterinspiegel verbessert.

4. Eine Methode entsprechend eines vorangegangenen Anspruchs, wobei GPR146 aus einem Polypeptid besteht oder dieses kodiert, das hierin als SEQ ID Nr. 4 oder SEQ ID-Nr. 6 oder als eine Sequenz mit mindestens 70% Seqzuenzidentität zu diesem identifiziert wird.

5. Eine Methode entsprechend eines vorangegangenen Anspruchs, wobei der GPR146 mit dem Kandidatenwirkstoff kontaktiert wird, um festzustellen, ob der Kandidatenwirkstoff die regulierende Wirkung von GPR146 auf den Serumcholesterinspiegel beeinträchtigt.

6. Eine Methode entsprechend eines vorangegangenen Anspruchs, wobei der Kandidatenwirkstoff mit einer Zelle kontaktiert wird, die ein GPR146-Polypeptid exprimiert.

7. Eine Methode entsprechend eines vorangegangenen Anspruchs, wobei der Schritt der Feststellung, ob der Wirkstoff die Wirkung von GPR146 beeinträchtigt, an einer Probe vorgenommen wird, die von einem nichtmenschlichen Tier erhalten wurde, das das GPR146-Polypeptid exprimiert und dem der Kandidatenwirkstoff verabreicht wurde, und von der Probe festgestellt wird, ob das Tier einen veränderten Lipidmetabolismus zeigt.

8. Eine Methode entsprechend Anspruch 7, wobei das nichtmenschliche Tier ein funktionelles GPR146-Polypeptid exprimiert.

9. Eine Methode entsprechend Anspruch 7 oder Anspruch 8, wobei es sich bei dem nichtmenschlichen Tier um einen Wildtyp handelt.

10. Eine Methode entsprechend einem der Ansprüche 7 bis 9, wobei es sich bei dem nichtmenschlichen Tier um ein Nagetier handelt.

11. Eine Methode entsprechend eines vorangegangenen Anspruchs, wobei es sich bei der Krankheit um Atherosklerose handelt.

12. Ein nichtmenschliches transgenes Tier mit einem funktionell gestörten endogenen GPR146-Gen, wobei das GPR146-Gen der Definition in Anspruch 1 entspricht.

13. Ein nichtmenschliches transgenes Tier entsprechend Anspruch 12, wobei das Tier eine Null-Mutation aufweist.

14. Ein nichtmenschliches transgenes Tier entsprechend Anspruch 12 oder Anspruch 13, wobei das Tier für das GPR146-Gen -/- ist.

15. Eine Methode zur Bestimmung des Vorhandenseins von oder einer Prädisposition für eine Krankheit gemäß der Definition in Anspruch 1 in einem Individuum, bestehend aus den folgenden Schritten, nämlich (i) Bestimmen des Expressionslevel des GPR146-Gens in einer vom Patienten isolierten Probe und (ii) Bestimmen, im Vergleich mit einer Kontrolle, ob das Individuum, von dem die Probe isoliert wurde, eine Krankheit gemäß der Definition in Anspruch hat oder für diese prädisponiert ist, wobei das GPR146-Gen der Definition in Anspruch 1 entspricht.

16. Verwendung eines exogenen GPR146-Polynukleotids gemäß der Definition in Anspruch 1 bei der Herstellung eines Medikaments zur Behandlung von oder Vorbeugung gegen eine in Anspruch 1 definierte Krankheit.

## Revendications

1. Procédé *in vitro* ou *ex vivo* d'identification d'un agent approprié à la thérapie d'un trouble du métabolisme des lipides sélectionné parmi : maladie athéromateuse, athérosclérose, cardiopathie ischémique, maladie de la vésicule biliaire, altération de la tolérance au glucose, syndrome métabolique X, resténose, choc septique, accident cardiovasculaire, angine de poitrine, attaques ischémiques transitoires (AITs) ou maladie des artères périphériques (MAP), comprenant les étapes de détermination si un agent candidat affecte l'activité de contrôle du taux de cholestérol sérique du GPR146, dans lequel le GPCR146 contient ou code un polypeptide identifié dans les présentes comme étant propre à la séquence SEQ ID No. 4 ou à la séquence SEQ ID No. 6 ou à une séquence ayant une identité de séquence d'au moins 70% par rapport à celles-ci.

2. Procédé selon la revendication 1, dans lequel l'agent est un antagoniste qui inhibe l'activité de contrôle du taux du cholestérol sérique du GPCR146.

3. Procédé selon la revendication 1, dans lequel l'agent est un agoniste qui améliore l'activité de contrôle du taux de cholestérol sérique du GPCR146.

4. Procédé selon l'une quelconque des revendications précédentes, dans lequel le GPR146 consiste en ou code un polypeptide identifié dans les présentes comme étant propre à la séquence SEQ ID No. 4, à la séquence SEQ ID No 6 ou à une séquence ayant une identité de séquence d'au moins 70% par rapport à celles-ci.

5. Procédé selon l'une quelconque des revendications précédentes, dans lequel le polypeptide du GPR146 est mis en contact avec l'agent candidat afin de déterminer si le candidat affecte l'activité de contrôle du taux de cholestérol sérique du GPCR146.

6. Procédé selon l'une quelconque des revendications précédentes, dans lequel l'agent candidat est mis en contact avec une cellule exprimant un polypeptide du GPCR146.

7. Procédé selon l'une quelconque des revendications précédentes dans lequel l'étape de détermination si l'agent affecte l'activité du GP146 est réalisée sur un échantillon pouvant être obtenu d'un animal non humain exprimant le polypeptide du GPR146 ayant été administré avec l'agent candidat et la détermination à partir de l'échantillon si l'animal présente un métabolisme des lipides altéré.

8. Procédé selon la revendication 7, dans lequel l'animal non humain exprime un polypeptide fonctionnel du GPCR146.

9. Procédé selon la revendication 7 ou la revendication 8, dans lequel l'animal non humain est de type sauvage.

10. Procédé selon l'une quelconque des revendications 7 à 9, dans lequel l'animal non humain est un rongeur.

11. Procédé selon l'une quelconque des revendications précédentes, dans lequel le trouble est l'athérosclérose.

12. Animal transgénique non humain ayant un gène endogène GPCR146 fonctionnellement perturbé, dans lequel le gène GPCR146 est tel que défini dans la revendication 1.

13. Animal transgénique non humain selon la revendication 12, dans lequel l'animal présente une mutation nulle.

14. Animal transgénique non humain selon la revendication 12 ou la revendication 13, dans lequel l'animal est -/- pour le gène GPCR146.

15. Procédé de détermination de la présence de, ou de la prédisposition à, un trouble défini dans la revendication 1 chez un individu, comprenant les étapes de (i) détermination du niveau d'expression du gène GPR146 dans un échantillon isolé du patient, et (ii) détermination comparée à un témoin, si l'individu à partir duquel l'échantillon a été isolé a ou est prédisposé à un trouble défini dans la revendication 1, dans lequel le gène GPCR146 est tel que défini à la revendication 1.

16. Utilisation d'un polypeptide exogène du GPR146 tel que défini dans la revendication 1 dans la fabrication d'un médicament destiné au traitement ou à la prévention d'un trouble défini dans la revendication 1.
